# EUROPÄISCHE PATENTSCHRIFT

(11) **EP 0 641 838 B1**
(45) Veröffentlichungstag und Bekanntmachung des Hinweises auf die Patenterteilung: **10.11.1999**
(21) Anmeldenummer: 94112968.6
(22) Anmeldetag: 19.08.1994
(51) Int. Cl.: C09B 62/006, D06P 1/382

(54) **Reaktivfarbstoffe auf Basis einer substituierten Barbitursäure**
Reactive dye-stuffs on basis of a substituted barbituric acid
Colorants réactifs à base d'un acide barbiturique substitué

(30) Priorität: 01.09.1993 DE 4329421
(43) Veröffentlichungstag der Anmeldung: 08.03.1995
(73) Patentinhaber: BAYER AG, 51368 Leverkusen (DE)
(72) Erfinder: Ehrenberg, Stefan, Dr., D-50670 Köln (DE); Engel, Aloysius, Dr., D-51381 Leverkusen (DE); Henk, Hermann, Dr., D-51061 Köln (DE)

(56) Entgegenhaltungen:
- DE-B- 1 239 038
- US-A- 4 540 776
- PATENT ABSTRACTS OF JAPAN, unexamined application, C Field, Band 9, Nr. 88 17. April 1985 THE PATENT OFFICE JAPANESE GOVERNMENT Seite 24 C 276; & JP-A-59 217 767 (SUMITOMO KAGAKU KOGYO K.K.)

## Beschreibung

Die Erfindung betrifft neue faserreaktive Azofarbstoffe auf Basis Barbitursäure, ihre Herstellung, Verwendung sowie neue Zwischenprodukte.

Faserreaktive Azofarbstoffe, bei denen die Kupplungs- oder die Azokomponente ein Heterocyclus ist, sind bereits bekannt. EP-A-13 879 (US-A-4 540 776) bezieht sich auf Azofarbstoffe, bei denen die Kupplungskomponente ein Pyrimidon aber auch unsubstituierte Barbitursäure sein kann. Das Schweizer Patent 489 582 (US-A-3 697 500) bezieht sich auf wasserlösliche Disazofarbstoffe, bei denen die Kupplungskomponente N-Methyl-N'-m-chlor-phenyl-barbitursäure sein kann. JP-A-5 9217-767 bezieht sich auf Azofarbstoffe, die als faserreaktive Gruppen ein Halogentriazin und ein Vinylsulfon bzw. Derivat davon und eine gegebenenfalls mit Alkyl oder Phenyl substituierte Barbitursäure aufweisen.

Die bekannten Verbindungen weisen Nachteile, insbesondere hinsichtlich Löslichkeit und Aufbauverhalten auf. Der Erfindung lag die Aufgabe zugrunde, Reaktivfarbstoffe zur Verfügung zu stellen, die insbesondere hinsichtlich Löslichkeit und Aufbauverhalten verbesserte Eigenschaften aufweisen.

Die vorliegende Erfindung betrifft faserreaktive Azofarbstoffe der Formel 1 und deren Tautomere 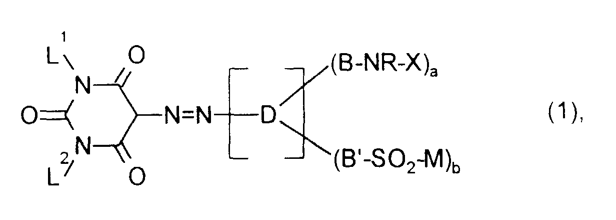 worin
- a: für 0, 1 oder 2,
- b: für 0, 1 oder 2 und
- a + b: für 1 oder 2 steht,
- D =: Rest einer gegebenenfalls substituierten aromatischen oder heterocyclischen Diazokomponente bedeutet, die gegebenenfalls eine Azogruppe enthält
- B,B' =: unabhängig voneinander direkte Bindung oder Brückenglied bedeutet und insbesondere mit einem Ring C-Atom eines aromatisch carbocyclischen oder heterocyclischen Ringes in D verknüpft ist,
- X =: faserreaktiver heterocyclischer Rest,
- R =: H, gegebenenfalls substituiertes C₁-C₄-Alkyl, wobei als Substituenten bevorzugt zu nennen sind Halogen, insbesondere Cl und F, OH, COOH, SO₃H und/oder OSO₃H,
- M=: CH₂-CH₂-OH, CH=CH₂ oder CH₂-CH₂-V worin V = alkalisch eliminierbarer Rest, bevorzugt OSO₃H, SSO₃H, OCOCH₃, OPO₃H₂, OSO₂CH₃, SCN, NHSO₂CH₃, Cl, Br, F, OCOC₆H₅, OSO2-C₆H₅, ^{⊕}N(CH₃)₃-Anion (Anion ist bevorzugtCl⁻)
- L¹, L² =: gleich oder verschieden, H oder eine aliphatische oder aromatische Gruppe, wobei wenigstens einer der Substituenten L¹ und L² mit wenigstens einem polaren Rest, wie z.B. OH, COOH, SO₃H, OSO₃H oder Alkoxy substituiert ist oder für Hydroxy steht.

Neben Sulfonsäuregruppen kann D bevorzugt Alkylgruppen mit 1 bis 4 Kohlenstoffatomen, wie Methyl, Ethyl, Propyl, Isopropyl, Butyl, Isobutyl, sek.-Butyl und tert.-Butyl, Alkoxygruppen mit 1 bis 4 Kohlenstoffatomen, wie Methoxy, Ethoxy, Propyloxy, Isopropyloxy, Butyloxy, Isobutyloxy, sek.-Butyloxy und tert.-Butyloxy, Phenoxy, Acylaminogruppen mit 1 bis 6 Kohlenstoffatomen, wie Acetylamino, Propionylamino und Benzoylamino, Aminogruppen, wie -NH₂-, Methylamino, Ethylamino und Phenylamino; Carbonsäureestergruppen, wie Methoxycarbonyl und Ethoxycarbonyl, die Nitro-, Cyan-, Acetyl-, Carbamoyl-, Ureido-, Hydroxy- und Carboxygruppe und Halogen, wie Fluor, Chlor und Brom enthalten.

Bevorzugt sind Farbstoffe der Formel 1, worin D ein Rest der Formel (2) bzw. deren Tautomeren 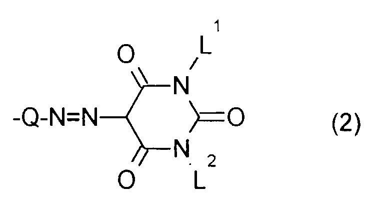 bedeutet,
mit Q = 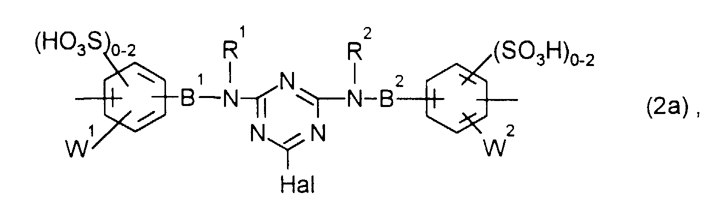 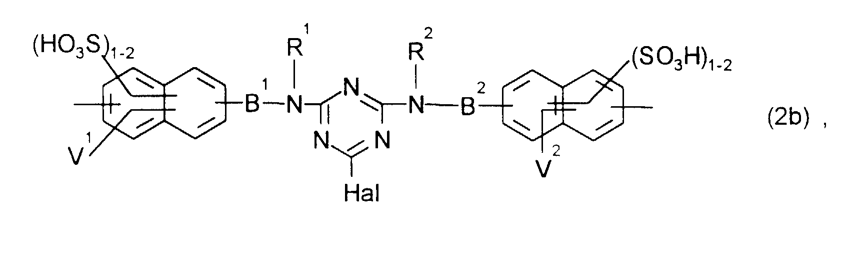 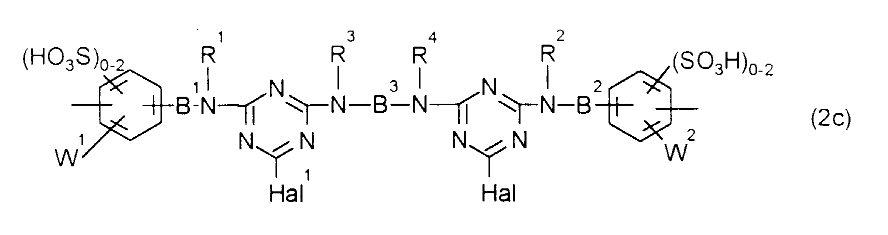 oder
Q = 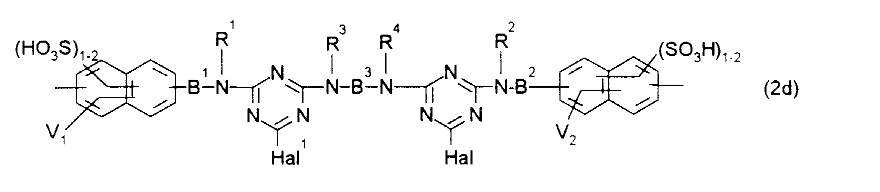 worin L¹ und L² die oben angegebenen Bedeutungen haben, und worin bedeuten
- V¹, V², W¹, W² =: unabhängig voneinander Wasserstoff oder die für D oben angegebenen Substituenten bedeuten, insbesondere C₁-C₄-Alkyl, C₁-C₄-Alkoxy, Acetylamino, NH₂, Methylamino, Ethylamino, Methoxycarbonyl, Ethoxycarbonyl, Nitro, Cyan, Acetyl, Carbamoyl, Ureido, Hydroxy, Carboxy, F, Cl oder Br,
- Hal, Hal¹ =: unabhängig voneinander Cl, F, Pyridinium oder substituiertes Pyridinyl,
- R¹,R²,R³,R⁴=: unabhängig voneinander H oder gegebenenfalls substituiertes C₁-C₄-Alkyl bedeutet, wobei als Substituenten bevorzugt Halogen, OH, COOH, SO₃H oder OSO₃H zu nennen sind,
- B und B': die obengenannte Bedeutung haben und
- B¹, B², B³ =: unabhängig voneinander eine direkte Bindung oder ein Brükkenglied folgender Formeln 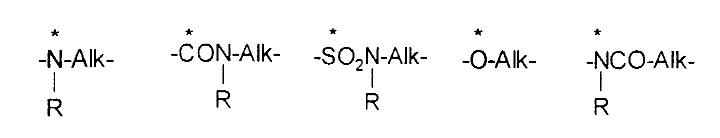 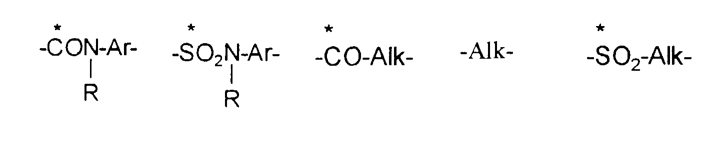
wobei der Stern die Verknüpfungsstelle mit einem Benzol oder Naphthalinrest markiert und worin
- Alk: gegebenenfalls durch Heteroatome oder Heteroatome wie N, O oder S enthaltende Gruppierungen unterbrochenes, geradkettiges oder verzweigtes C₁-C₆-Alkylen,
- Ar: gegebenenfalls substituiertes Phenylen oder Naphthylen oder Rest eines Diphenyls oder Stilbens,
- T: Alk oder Ar oder 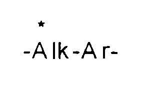 wobei Alk oder Ar gegebenenfalls mit F, Cl, Br, C₁-C₄-Alkyl, C₁-C₄-Alkoxy, Carboxy oder Sulfo weiter substituiert sind,
- E: F, Cl, Br, gegebenenfalls substituiertes Amino, OH, C₁-C₄-Alkoxy, gegebenenfalls substituiertes Phenoxy, C₁-C₄-Alkylthio bedeutet.

In einer besonderen Ausführungsform sind Farbstoffe der Formel 2a bis 2d zu nennen, worin
W¹ = W²,
V¹ = V²,
R¹ = R²,
Hal = Hal¹,
R³ = R⁴ und
B¹ = B² ist.

Weitere bevorzugte Farbstoffe der Formel (1) sind solche, worin
a = 0
und
-D-(B'-SO₂-M)_{b} einem Rest folgender Formel (3) entspricht 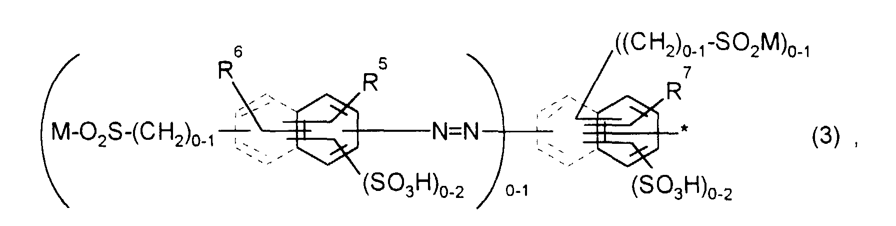 worin bedeuten
- R⁵: H, C₁-C₄-Alkyl, Cl, Br, C₁-C₄-Alkoxy oder COOH,
- R⁶: H, C₁-C₄-Alkyl, C₁-C₄-Alkoxy, SO₃H, Cl oder Br,
- R⁷: H, C₁-C₄-Alkyl, C₁-C₄-Alkoxy, Cl, Br, Acylamino, insbesondere C₁-C₄-Alkylcarbonylamino oder Arylcarbonylamino, wie gegebenenfalls substituiertes Phenylcarbonylamino, C₁-C₄-Alkylsulfonylamino, Aminocarbonylamino, Arylsulfonylamino,
- M: die obengenannte Bedeutung hat
und die mit * gekennzeichnete Bindung an die Azogruppe der Formel (1) gebunden ist.

Der Rest der Formel (3) leitet sich vorzugsweise von folgenden Diazokomponenten ab:
Anilin-4-β-sulfatoethylsulfon,
Anilin-4-β-thiosulfatoethylsulfon,
Anilin-4-vinylsulfon,
Anilin-3-β-sulfatoethylsulfon,
Anilin-3-vinylsulfon,
2-Methoxy-anilin-5-β-sulfatoethylsulfon,
2-Methoxy-anilin-5-β-thiosulfatoethylsulfon,
2-Methoxyanilin-5-vinylsulfon,
4-Methoxy-anilin-3-β-sulfatoethylsulfon,
4-Methoxy-anilin-3-β-vinylsulfon,
2,5-Dimethoxyanilin-4-β-sulfatoethylsulfon,
2,5-Dimethoxy-anilin-4-vinylsulfon,
2-Methoxy-5-methyl-anilin-4-β-sulfatoethylsulfon,
Anilin-2-β-sulfatoethylsulfon,
3-(3- oder 4-Aminobenzoyl)-aminophenyl-β-sulfatoethylsulfon,
2-Methoxy-5-methyl-anilin-4-vinylsulfon,
6-Carboxy-anilin-3-β-sulfatoethylsulfon,
6-Carboxyanilin-3-vinylsulfon,
2-Sulfoanilin-4-β-sulfatoethylsulfon,
2-Sulfoanilin-4-vinylsulfon,
2,4-Disulfoanilin-5-vinylsulfon,
2-Naphthylamin-8-β-sulfatoethylsulfon,
2-Naphthylamin-6-β-sulfatoethylsulfon,
1-Sulfo-2-naphthylamin-6-β-sulfatoethylsulfon,
1-Naphthylamin-4-β-sulfatoethylsulfon,
1-Sulfo-2-naphthylamin-5-β-sulfatoethylsulfon,
6-Sulfo-2-naphthylamin-8-β-sulfatoethylsulfon,
2-Amino-3-sulfo-naphthalin-6,8-bis-(β-sulfatoethylsulfon),
1-Naphthylamin-5-β-sulfatoethylsulfon,
2-Naphthylamin-5-β-sulfatoethylsulfon,
2-Naphthylamin-8-β-sulfatoethylsulfon,
8-Sulfo-2-naphthylamin-6-β-sulfatoethylsulfon,
4-Aminobenzyl-β-sulfatoethylsulfon,
3 -Aminobenzyl-β-sulfatoethylsulfon,
4-Aminobenzylvinylsulfon,
3-Aminobenzylvinylsulfon,
3 -Amino-4-sulfobenzyl-β-sulfatoethylsulfon,
4-Amino-3-sulfobenzylvinylsulfon,
4-Vinylsulfonyl-butansäure-(4'-amino-3'-sulfo)-anilid,
3 -Amino-N-[2'-(β-sulfatoethylsulfonyl)-ethyl]-benzamid,
4-Vinyl sulfonyl-butansäure-(5'-amino-2',4'-disulfo)-anilid,
4-Amino-3 -methoxy-N-(2-vinylsulfonyl-ethyl)-benzamid,
4-(β-Sulfatoethylsulfonyl)-butansäure-(3-amino-4'-sulfo)-anilid,
4-(β-Chloroethylsulfonyl)-butansäure-(4'-amino-3'-sulfo)-anilid,
4-Amino-N-[2-vinylsulfonyl-ethyl]-benzamid,
3 -Vinylsulfonyl-propansäure-(4'-amino-2',5'-disulfo)-anilid,
2'-(β-Sulfatoethylsulfonyl)-3 -sulfo-4-aminoazobenzol,
3'-(β-Sulfatoethyl sulfonyl)-3-sulfo-4-aminoazobenzol,
4'-Methoxy-3'-(β-sulfatoethylsulfonyl)-3-sulfo-4-aminoazobenzol,
4'-Vinyl sulfonyl-2',3 -disulfo-4-aminoazobenzol,
2'-(β-Sulfatoethylsulfonyl)-6-methyl-3-sulfo-4-aminoazobenzol,
3'-(β-Sulfatoethylsulfonyl)-6-methyl-3-sulfo-4-aminoazobenzol,
4'-(β-Sulfatoethylsulfonyl)-6-methyl-3 -sulfo-4-aminoazobenzol,
4'-(β-Sulfatoethylsulfonyl)-2,6-dimethyl-3 -sulfo-4-aminoazobenzol,
3'-(β-Sulfatoethylsulfonyl)-6-methoxy-3 -sulfo-4-aminoazobenzol,
3',4'-Bis-(β-sulfatoethylsulfonyl)-6-methoxy-3 -sulfo-4-aminoazobenzol,
4'-(β-Sulfatoethyl sulfonyl)-6-methoxy-3 -sulfo-4-aminoazobenzol,
4'-(β-Sulfatoethyl sulfonyl)-2-methyl-5 -methoxy-3 -sulfo-4-aminoazobenzol,
4'-(β-Sulfatoethyl sulfonyl)-2,5 -dimethoxy-3 -sulfo-4-aminoazobenzol,
3'-(β-Sulfatoethylsulfonyl)-2,5-dimethoxy-3 -sulfo-4-aminoazobenzol,
2-(4'-Amino-3'-sulfophenylazo)-6-(β-sulfatoethylsulfonyl)-naphthalin,
2-(4'-Amino-6'-methyl-3'-sulfophenylazo)-1-sulfo-6-(β-sulfatoethyl-sulfonyl)-naphthalin,
2-(4'-Amino-6'-methyl-3-sulfophenylazo)-6-(β-sulfatoethylsulfonyl)-naphthalin,
2-(4'-Amino-3'-sulfophenylazo)-8-sulfo-6-(β-sulfatoethylsulfonyl)-naphthalin,
2-(4'-Amino-6'-methyl-3'-sulfophenylazo)-1,7-disulfo-5-(β-sulfatoethylsulfonyl)-naphthalin.

Ebenfalls bevorzugt sind solche Farbstoffe der Formel (1) und deren Tautomere, worin
b = 0,
a = 1 und die übrigen Substituenten die obigen Bedeutungen besitzen.

Insbesondere zu nennen sind dabei Farbstoffe der Formel (4) 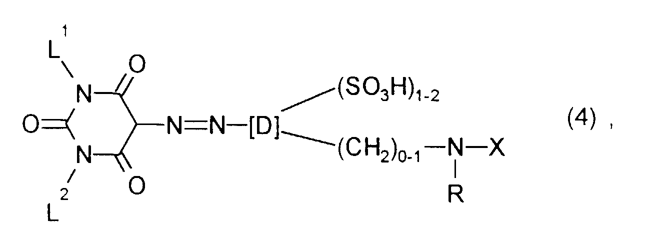 worin
- D =: Rest eines Benzols oder Naphthalins, das gegebenenfalls durch C₁-C₄-Alkyl, C₁-C₄-Alkoxy, CO₂H, 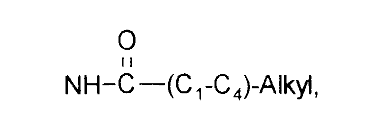 NH-CO-NH₂ oder Halogen, insbesondere Cl oder F substituiert ist
und L¹, L², R und X die obige Bedeutung besitzen.

Ebenfalls bevorzugt sind solche Farbstoffe der Formel (1) und deren Tautomeren, worin b = 0, a = 1 und D ein sulfonsäuregruppenhaltiger Rest einer heterocyclischen Diazokomponente bedeutet, der gegebenenfalls substituiert ist, vorzugsweise durch C₁-C₄-Alkyl, C₁-C₄-Alkoxy, 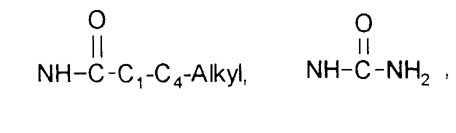 COOH oder Halogen.

Besonders bevorzugt sind dabei Farbstofe der Formel (5) 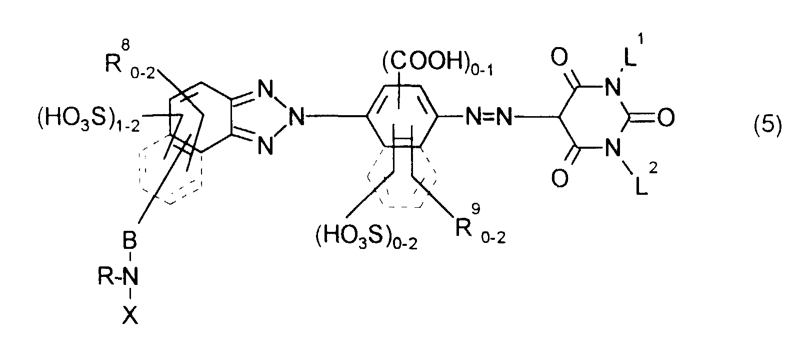 insbesondere solche der Formel (5a) 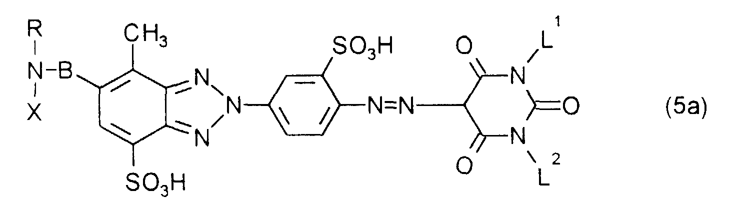 und (5b) 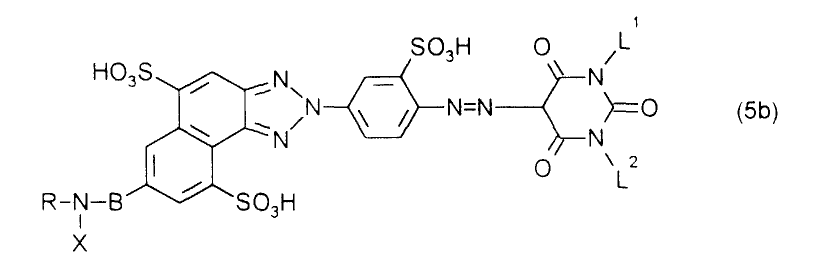 worin R⁸ und R⁹ unabhängig voneinander vorzugsweise Wasserstoff, C₁-C₄-Alkyl, C₁-C₄-Alkoxy, Acetylamino, NH₂, Methylamino, Ethylamino, Carbamoyl, Ureido, Hydroxy und Acetyl sein können und die übrigen Substituenten die unter Formel (1) angegebene Bedeutung haben.

Ebenfalls bevorzugt sind Farbstoffe der Formel (6) 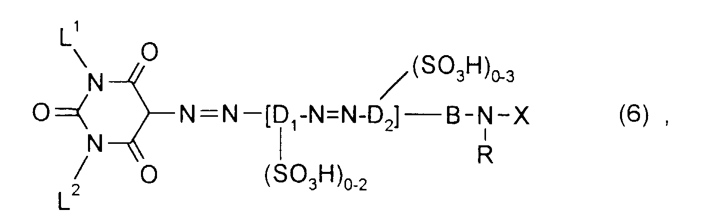 worin
- D₁ und D₂: unabhängig voneinander einen Phenylen- oder Naphthylrest, der durch C₁-C₄-Alkyl, C₁-C₄-Alkoxy, CO₂H, NHCO-C₁-C₄-Alkyl, NHCONH₂ oder Halogen insbesondere Cl oder F substituiert sein kann, bedeuten und B, R und X die obengenannten Bedeutungen besitzen.

Besonders bevorzugte Farbstoffe der Formel (6) sind solche der Formeln (6a), (6b) und (6c) 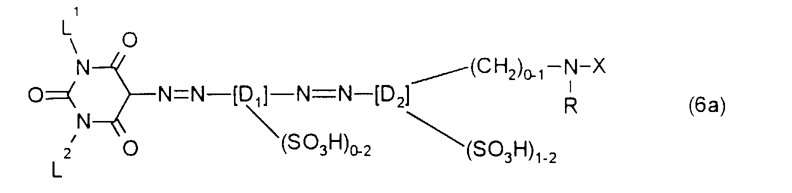 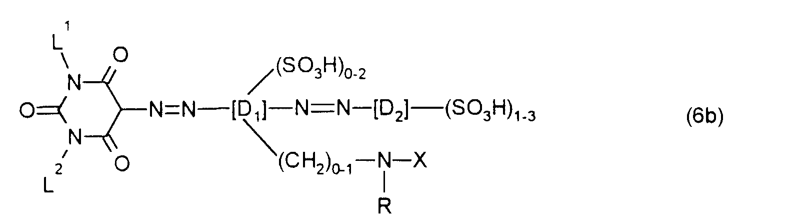 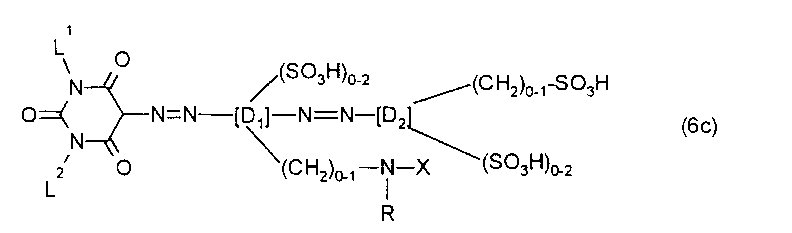 worin D₁, D₂, R und X die obigen Bedeutungen besitzen.

Weiterhin sind Farbstoffe der Formel (1) bevorzugt, die der Forme (7) entsprechen 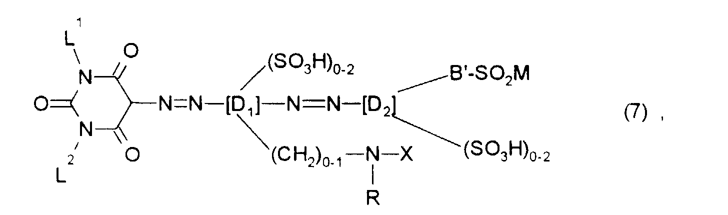 worin
- D₁ und D₂: unabhängig voneinander einen Phenylen- oder Naphthylenrest, der durch C₁-C₄-Alkyl, C₁-C₄-Alkoxy, CO₂H, 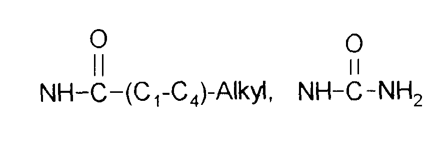 oder Halogen, insbesondere Cl oder F, substituiert sein kann, bedeuten
und
L¹, L², M, B', R und X die obigen Bedeutungen besitzen.

Besonders bevorzugte Farbstoffe der Formel (1) sind solche, worin bedeuten:
- L¹ =: 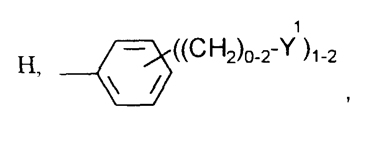 -(CH₂)₁₋₅-Y²,
- L² =: 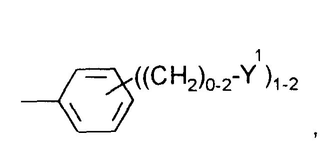 -(CH₂)₁₋₅-Y², -OH,
- Y¹ =: unabhängig voneinander bei L¹ und L²:
OH, COOH, SO₃H, C₁-C₄-Alkoxy, Hydroxy-C₁₋₄-Alkoxy,
HO-(CH₂-CH₂-O)₁₋₃-, HOOC-(CH₂-CH₂-O)₁₋₃-, HO₃S-(CH₂-CH₂-O)₁₋₃-, HO₃S-CH₂-CH₂-CH₂-O-, HOOC-CH₂-CH₂-CH₂-O-,
- Y² =: unabhängig voneinander bei L¹ und L²:
OH, COOH, OSO₃H, SO₃H, C₁-C₄-Alkoxy, Hydroxy-C₁₋₄-Alkoxy, HO-(CH₂-CH₂-O)₁₋₃-, HOOC-(CH₂-CH₂-O)₁₋₃-, HO₃S-(CH₂-CH₂-O)₁₋₃-, HO₃S-CH₂-CH₂-CH₂-O-, HOOC-CH₂-CH₂-CH₂-O-,

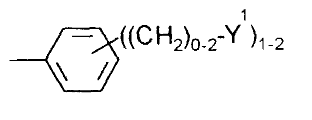

Ganz besonders bevorzugt sind Farbstoffe der Formel 1, worin bedeuten:
- L¹ =: H, 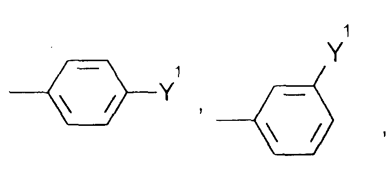 -(CH₂)₁₋₅-Y²,
- L² =: 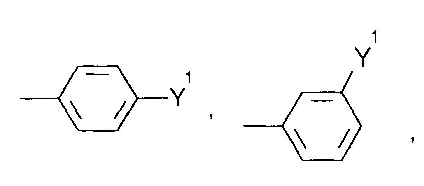 -(CH₂)₁₋₅-Y²,
- Y² =: OH, OSO₃H, COOH, SO₃H, 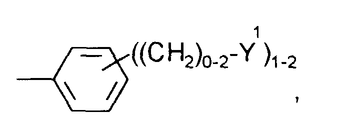
- Y¹ =: OH, COOH, SO₃H.

Bevorzugte Bedeutungen für D, D₁ und D₂ leiten sich beispielsweise von folgenden Diazokomponenten ab:

1,3-Diaminobenzol, 1,4-Diaminobenzol, 1,3-Diamino-4-chlorbenzol, 1,3-Diamino-4-methylbenzol, 1,3-Diamino-4-ethylbenzol, 1,3-Diamino-4-methoxybenzol, 1,3-Diamino-4-ethoxybenzol, 1,4-Diamino-2-methylbenzol, 1,4-Diamino-2-methoxybenzol, 1,4-Diamino-2-ethoxybenzol, 1,4-Diamino-2-chlorbenzol, 1,4-Diamino-2,5-dimethylbenzol, 1,4-Diamino-2,5-diethylbenzol, 1,4-Diamino-2-methyl-5-methoxybenzol, 1,4-Diamino-2,5-dimethoxybenzol, 1,4-Diamino-2,5-diethoxybenzol, 2,6-Diamino-naphthalin, 1,3-Diamino-2,4,6-trimethylbenzol, 1,4-Diamino-2,3,5,6-tetramethylbenzol, 1,3-Diamino-4-nitrobenzol, 4,4'-Diaminostilben, 4,4'-Diaminodiphenylmethan, 4,4'-Diaminobiphenyl (Benzidin), 3,3'-Dimethylbenzidin, 3,3'-Dimethoxybenzidin, 3,3'-Dichlorbenzidin, 3,3'-Dicarboxybenzidin, 3,3'-Dicarboxymethoxy-benzidin, 2,2'-Dimethylbenzidin, 4,2'-Diaminodiphenyl (Diphenylin), 2,6-Diaminonaphthalin-4,8-disulfonsäure, 1,4-Diaminobenzol-2-sulfonsäure, 1,4-Diaminobenzol-2,5-disulfonsäure, 1,4-Diaminobenzol-2,6-disulfonsäure, 1,3-Diaminobenzol-4-sulfonsäure, 1,3-Diaminobenzol-4,6-disulfonsäure, 1,4-Diamino-2-chlorbenzol-5-sulfonsäure, 1,4-Diamino-2-methylbenzol-5-sulfonsäure, 1,5-Diamino-6-methylbenzol-3-sulfonsäure, 1,3-Diamino-6-methylbenzol-4-sulfonsäure, 3-(3'-bzw. 4'-Aminobenzoylamino)-1-aminobenzol-6-sulfonsäure, 1-(4'-Aminobenzoylamino)-4-aminobenzol-2,5-disulfonsäure, 1,4-Diaminobenzol-2-carbonsäure, 1,3-Diaminobenzol-4-carbonsäure, 1,3-Diaminobenzol-4,6-disulfonsäure, 1,3-Diaminobenzol-5-carbonsäure, 1,4-Diaminobenzol-2-methylbenzol, 4,4'-Diaminodiphenyloxid, 4,4'-Diaminodiphenylharnstoff-2,2'-disulfonsäure, 4,4'-Diaminodiphenyl oxyethan-2,2'-disulfonsäure, 4,4'-Diaminostilben-2,2'-disulfonsäure, 4,4'-Diaminodiphenylethan-2,2'-disulfonsäure, 2-Amino-5-aminomethylnaphthalin-1-sulfonsäure, 1-sulfonsäure, 2-Amino-5-aminomethylnaphthalin- 1,7-disulfonsäure, 1-Amino-4-methoxy-5-aminomethylbenzol-6-sulfonsäure, 1-Amino-4-methyl-5-aminomethylbenzol-6-sulfonsäure.

Geeignete der Formel 1 zugrundeliegende Kupplungskomponenten 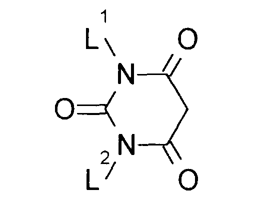 können bevorzugt sein:
1-Carboxymethylbarbitursäure
1-(2-Carboxyethyl)barbitursäure
1 -(3 -Carboxypropyl)barbitursäure
1-(5-Carboxypentyl)barbitursäure
1 -(2-Hydroxyethyl)barbitursäure
1-(3 -Hydroxypropyl)barbitursäure
1-(4-Hydroxybutyl)barbitursäure
1-Sulfomethylbarbitursäure
1-(2-Sulfoethyl)barbitursäure
1-(3-Sulfophenyl)barbitursäure
1-(4-Sulfophenyl)barbitursäure
1,3-Bis-(2-hydroxyethyl)barbitursäure
1,3-Bis-(carboxymethyl)barbitursäure
1,3-Bis-(2-carboxyethyl)barbitursäure
1,3-Bis-(3-sulfophenyl)barbitursäure
1 -Carboxymethyl-3 -(2-hydroxyethyl )barbitursäure
1-(3-Sulfopropyl)barbitursäure
1-(2-Sulfatoethyl)barbitursäure
1,3-Bis-(2-sulfatoethyl)barbitursäure

Bevorzugte Bedeutungen für B und B' sind unabhängig voneinander eine direkte Bindung oder ein Brückenglied der Formeln 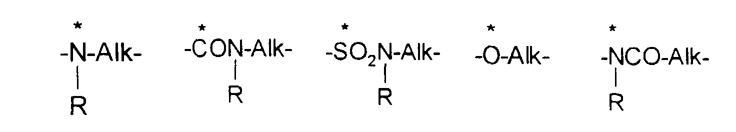 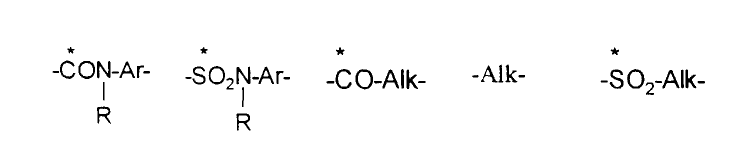 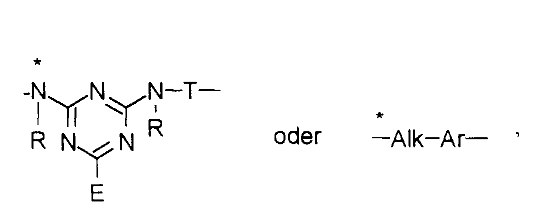 wobei der Stern die Verknüpfungsstelle mit einem Benzol oder Naphthalinrest markiert ist und worin
- Alk: gegebenenfalls durch Heteroatome oder Heteroatome wie N, O oder S enthaltende Gruppierungen unterbrochenes, geradkettiges oder verzweigtes C₁-C₆-Alkylen,
- Ar: gegebenenfalls substituiertes Phenylen oder Naphthylen oder Rest eines Diphenyls oder Stilbens,
- T: Alk oder Ar oder 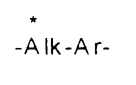 wobei Alk oder Ar gegebenenfalls mit F, Cl, Br, C₁-C₄-Alkyl, C₁-C₄-Alkoxy, Carboxy oder Sulfo substituiert sind,
- E: F, Cl, Br, gegebenenfalls substituiertes Amino, OH, C₁-C₄-Alkoxy, gegebenenfalls substituiertes Phenoxy, C₁-C₄-Alkylthio bedeutet.

Geeignete faserreaktive Reste X, d.h. solche die mit den OH- oder NH-Gruppen der Faser unter Färbebedingungen unter Ausbildung kovalenter Bindungen reagieren, sind insbesondere solche, die mindestens einen reaktiven Substituenten an einen 5- oder 6-gliedrigen aromatisch-heterocyclischen Ring gebunden enthalten, beispielsweise an einen Monoazin-, Diazin- oder Triazinring, insbesondere einen Pyridin-, Pyrimidin-, Pyridazin-, Pyrazin-, Thiazin-, Oxazin- oder asymmetrischen oder symmetrischen Triazinring, oder an ein derartiges Ringsystem, welches einen oder mehrere ankondensierte aromatisch-carbocyclische Ringe aufweist, beispielsweise ein Chinolin-, Phthalazin-, Cinnolin-, Chinazolin-, Chinoxalin-, Acridin-, Phenazin- und Phenanthridin-Ring-System.

Besonders geeignet sind dabei faserreaktive Reste aus der Reihe Pyrimidine oder Triazine.

Unter den reaktiven Substituenten am Heterocyclus sind beispielsweise zu erwähnen Halogen (Cl, Br oder F), Ammonium einschließlich Hydrazinium, Pyridinium, Picolinium, Carboxypyridinium, Sulfonium, Sulfonyl, Azido(N₃), Rhodanido, Thiolether, Oxiether, Sulfinsäure und Sulfonsäure.

Insbesondere kann X demnach stehen für 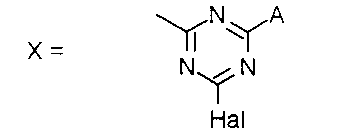 worin
- Hal =: Cl, F
- A =: Rest eines Amins AH, wobei bevorzugte Verbindungen AH sind:
Ammoniak, Methylamin, Ethylamin, n-Propanolamin, iso-Propanolamin, n-Butylamin, iso-Butylamin, tert.-Butylamin, n-Pentylamin, n-Hexylamin, Cyclohexylamin, Dimethylamin, Diethylamin, Di-n-propylamin, Di-iso-propylamin, Methylethylamin, Ethanolamin, Diethanolamin, 2-Methoxyethylamin, 2-Ethoxyethylamin, Sulfatoethylamin, Aminoessigsäure, N-Methylaminoessigsäure, Taurin, N-Methyltaurin, Methylaminomethansulfonsäure, Pyrrolidin, Piperidin, 1-Methyl-piperazin, Morpholin, Benzylamin, β-Phenylethylamin, N-Methylbenzylamin, Dibenzylamin, Anilin,
1-Amino-2-, 2- oder 4-methylbenzol,
1-Amino-3,4- oder 3,5-dimethylbenzol,
1-Amino-2-, 3- oder 4-ethylbenzol,
1-Amino-2-, 3- oder 4-methoxybenzol,
1-Amino-4-ethoxybenzol,
1-Amino-2-, 3- oder 4-(2-hydroxyethoxy)-benzol,
1-Amino-2-, 3- oder 4-(2-methoxyethoxy)-benzol,
1-Amino-2-, 3- oder 4-chlorbenzol,
2-, 3- oder 4-Amino-phenylmethansulfonsäure,
2-Aminobenzolsulfonsäure,
3-Aminobenzolsulfonsäure,
4-Aminobenzolsulfonsäure,
5-Aminobenzol-1,3- oder 1,4-disulfonsäure,
4-Aminobenzol-1,2- oder 1,3-disulfonsäure,
2-, 3- oder 4-Aminobenzolsulfonamid,
2-, 3- oder 4-Aminobenzolsulfonsäuremethylamid,
2-, 3- oder 4-Aminobenzolsulfonsäuredimethylamid,
2-, 3- oder 4-Aminobenzolsulfonsäure-(2-hydroxyethyl)-amid,
5-Aminobenzol-1,3-dicarbonsäure,
2-, 3- oder 4-Aminobenzoesäure,
2-, 3- oder 4-Aminobenzamid,
2-, 3- oder 4-Aminobenzoesäuremethyl- oder ethylester,
2-, 3- oder 4-Aminobenzonitril,
3-Amino-(N-phenylsulfonyl)-benzolsulfonamid,
2-, 3- oder 4-Aminophenol,
5-Amino-2-hydroxybenzolsulfonsäure,
4-Amino-2-hydroxybenzolsulfonsäure,
5-Amino-2-ethoxybenzolsulfonsäure,
1-Acetylamino-2- oder 4-aminobenzol,
1-Amino-3- oder 4-(hydroxyacetyl)-aminobenzol,
1-Amino-4-(sulfoacetyl)-aminobenzol,
3- oder 4-Aminphenylharnstoff,
N-(3-Aminophenyl)-N'-(2-hydroxyethyl)-harnstoff,
3- oder 4-Aminophenyloxymidsäure,
1-Methylamino-3- oder 4-methylbenzol,
1-Ethylamino-4-chlorbenzol,
2-Amino-5-methoxy-benzolsulfonsäure,
3 Amino-4-methoxy-benzolsulfonsäure,
1-Ethylamino-3- oder 4-methylbenzol,
N-(2-Hydroxyethyl)-anilin,
1-(2-Hydroxyethyl)-amino-3-methylbenzol,
3- oder 4-Methylaminobenzoesäure,
4-Methylaminobenzolsulfonsäure,
5-Amino-2-oxalamino-benzolsulfonsäure,
2-Aminonaphthalin-1-sulfonsäure,
4-Aminonaphthalin-1-sulfonsäure,
5-Aminonaphthalin-1-sulfonsäure,
6-Aminonaphthalin-1-sulfonsäure,
7-Aminonaphthalin-1-sulfonsäure,
8-Aminonaphthalin-1-sulfonsäure,
1-Aminonaphthalin-2-sulfonsäure,
4-Aminonaphthalin-2-sulfonsäure,
5-Aminonaphthalin-2-sulfonsäure,
6-Aminonaphthalin-2-sulfonsäure,
7-Aminonaphthalin-2-sulfonsäure,
7-Methylaminonaphthalin-2-sulfonsäure,
7-Butylaminonaphthalin-2-sulfonsäure,
7-Isobutylaminonaphthalin-2-sulfonsäure,
8-Aminonaphthalin-2-sulfonsäure.
4-Aminonaphthalin-1,3-disulfonsäure,
5-Aminonaphthalin-1,3-disulfonsäure,
6-Aminonaphthalin-1,3-disulfonsäure,
7-Aminonaphthalin-1,3-disulfonsäure,
8-Aminonaphthalin-1,3-disulfonsäure,
2-Aminonaphthalin-1,5-disulfonsäure,
3-Aminonaphthalin-1,5-disulfonsäure,
4-Aminonaphthalin-1,5-disulfonsäure,
4-Aminonaphthalin-1,6-disulfonsäure,
8-Aminonaphthalin-1,6-disulfonsäure,
4-Aminonaphthalin-1,7-disulfonsäure,
3-Aminonaphthalin-2,6-disulfonsäure,
4-Aminonaphthalin-2,6-disulfonsäure,
3-Aminonaphthalin-2,7-disulfonsäure,
4-Aminonaphthalin-2,7-disulfonsäure,
6-Aminonaphthalin-1,3,5-trisulfonsäure,
7-Aminonaphthalin-1,3,5-trisulfonsäure,
8-Aminonaphthalin-1,3,5-trisulfonsäure,
4-Aminonaphthalin-1,3,6-trisulfonsäure,
7-Aminonaphthalin-1,3,6-trisulfonsäure,
8-Aminonaphthalin-1,3,6-trisulfonsäure,
4-Aminonaphthalin-1,3,7-trisulfonsäure,

Der Rest X ist bevorzugt ein Halogentriazinylrest, der auch mit einem zweiten Halogentriazinylrest oder einem Halogendiazinylrest oder einem oder mehreren Vinylsulfonyl- oder Sulfatoethylsulfonylresten direkt oder über ein Brückenglied verknüpft sein kann. Entsprechende Brückenglieder sind bevorzugt: 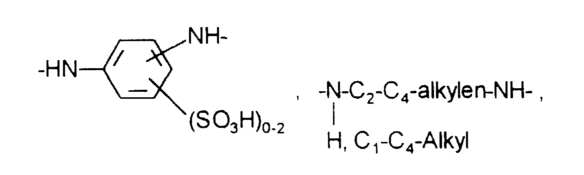 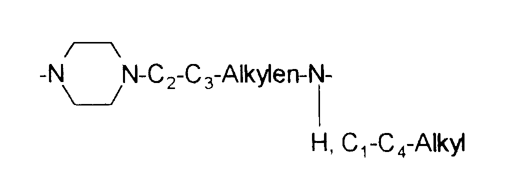 oder im Falle der Sulfatoethylsulfonyl- bzw. Vinylsulfonylgruppe über ein Brückenglied

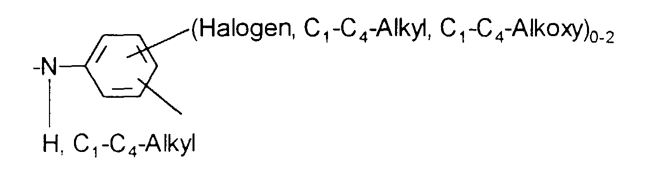

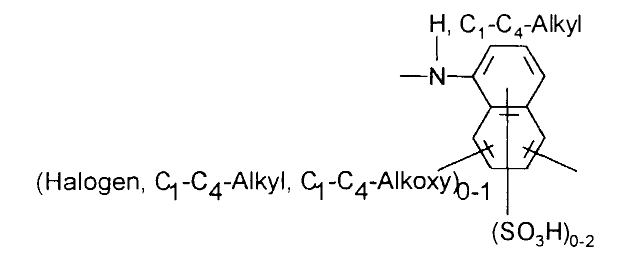

Hierbei können die Alkylreste ihrerseits substituiert sein, insbesondere mit SO₃H, COOH, OH, OSO₃H.

Beispiele für derartige Reste X sind:

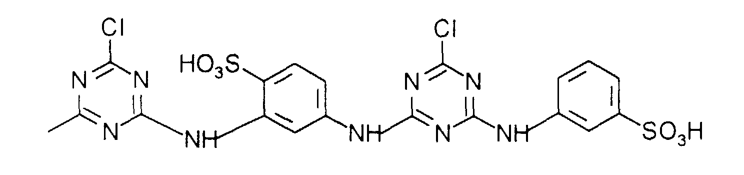

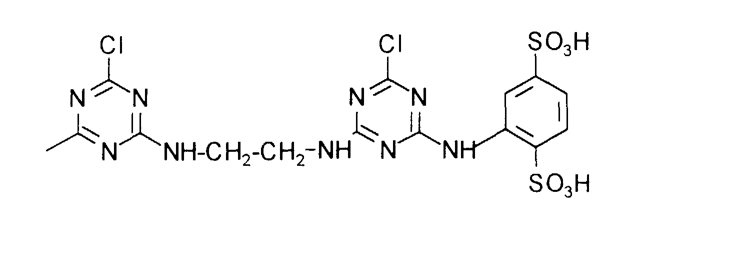

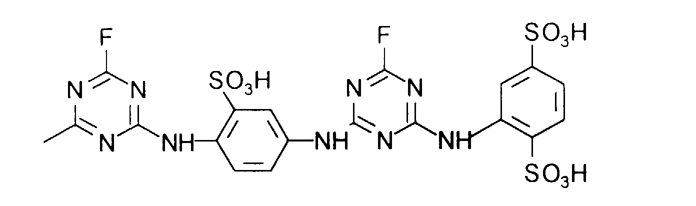

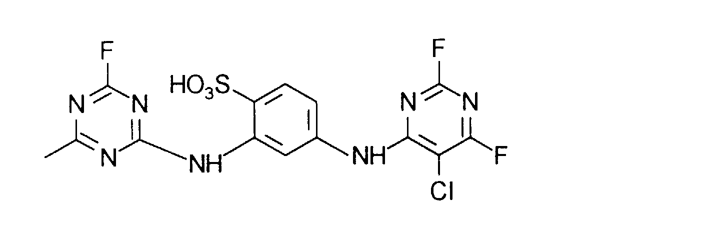

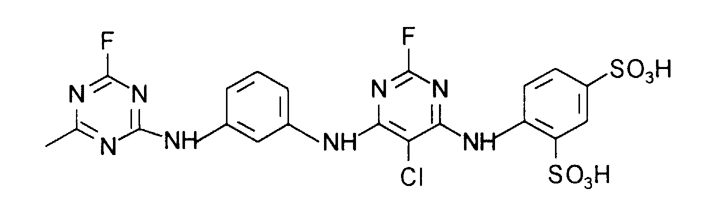

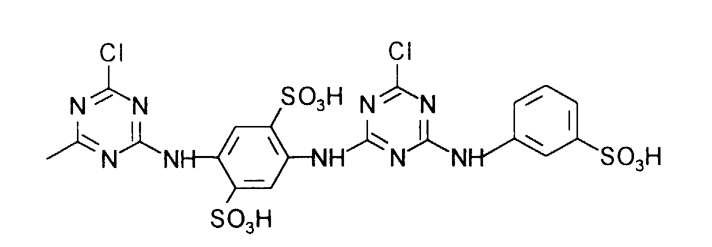

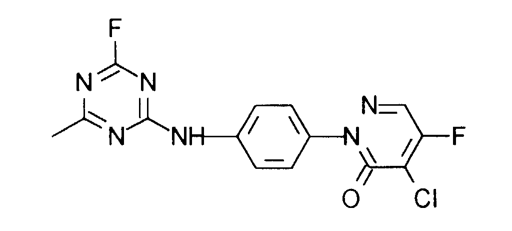

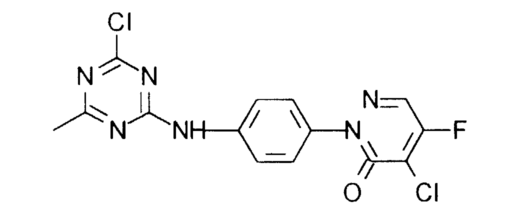

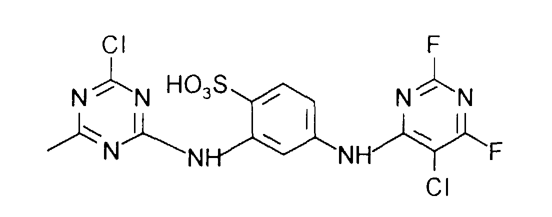

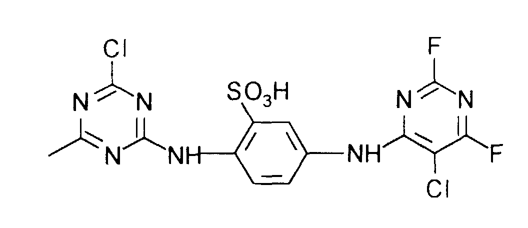

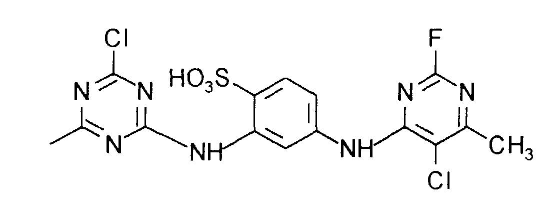

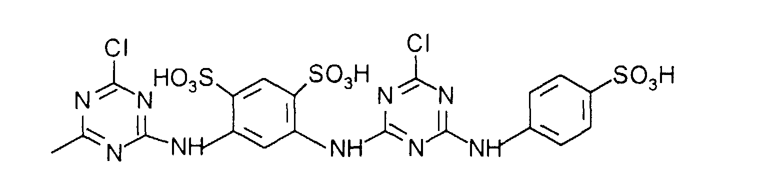

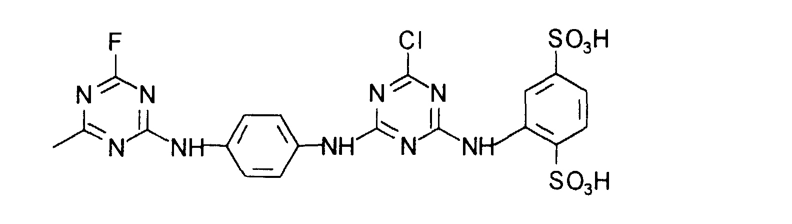

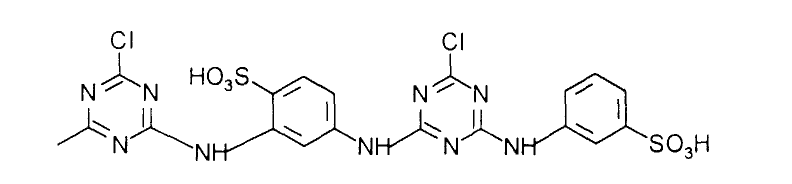

Weitere Beispiele für X sind:

Mono-, Di- oder Trihalogenpyrimidinylreste, wie 2,4-Dichlorpyrimidinyl-6-, 2,4,5-Trichlorpyrimidinyl-6-, 2,4-Dichlor-5-nitro- oder -5-methyl- oder -5-carboxymethyl- oder -5-carboxy- oder -5-cyano- oder -5-vinyl- oder -5-sulfo- oder -5-mono-, -di- oder -trichlormethyl- oder -5-carbalkoxy-pyrimidinyl-6-, 2,6-Dichlorpyrimidin-4-carbonyl-, 2,4-Dichlorpyrimidin-5-carbonyl-, 2-Chlor-4-methylpyrimidin-5-carbonyl-, 2-Methyl-4-chlorpyrimidin-5-carbonyl-, 2-Methylthio-4-fluorpyrimidin-5-carbonyl-, 6-Methyl-2,4-dichlorpyrimidin-5-carbonyl-, 2,4,6-Trichlorpyrimidin-5-carbonyl-, 2,4-Dichlorpyrimidin-5-sulfonyl-, 2-Chlor-chinoxalin-3-carbonyl-, 2- oder 3-Monochlorchinoxalin-6-carbonyl-, 2- oder 3-Monochlorchinoxalin-6-sulfonyl-, 2,3-Dichlorchinoxalin-5- oder -6-carbonyl-, 2,3-Dichlorchinoxalin-5- oder 6-sulfonyl-, 1,4-Dichlorphthalazin-6-sulfonyl- oder -6-carbonyl-, 2,4-Dichlorchinazolin-7- oder -6-sulfonyl- oder -carbonyl-, 2- oder 3- oder 4-(4',5'-Dichlorpyridazon-6'-yl-1')-phenylsulfonyl- oder -carbonyl-, β-(4',5'-Dichlorpyridazon-6'-yl-1')-ethylcarbonyl-, N-Methyl-N-(2,3-dichlorchinoxalin-6-sulfonyl)aminoacetyl-, N-Methyl-N-(2,3-dichlorchinoxalin-6-carbonyl)-aminoacetyl-, sowie die entsprechenden Brom- und Fluor-Derivate der oben erwähnten chlorsubstituierten heterocyclischen Reste, unter diesen beispielsweise 2-Fluor-4-pyrimidinyl-, 2,6-Difluor-4-pyrimidinyl-, 2,6-Difluor-5-chlor-4-pyrimidinyl-, 2-Fluor-5,6-dichlor-4-pyrimidinyl-, 2,6-Difluor-5-methyl-4-pyrimidinyl-, 2-Fluor-5-methyl-6-chlor-4-pyrimidinyl-, 2-Fluor-5-nitro-6-chlor-4-pyrimidinyl-, 5-Brom-2-fluor-4-pyrimidinyl-, 2-Fluor-5-cyan-4-pyrimidinyl-, 2-Fluor-5-methyl-4-pyrimidinyl-, 2,5,6-Trifluor-4-pyrimidinyl-, 5-Chlor-6-chlormethyl-2-fluor-4-pyrimidinyl-, 5-Chlor-6-dichlormethyl-2-fluor-4-pyrimidinyl-, 5-Chlor-6-trichlormethyl-2-fluor-4-pyrimidinyl-, 5-Chlor-2-chlormethyl-6-fluor-4-pyrimidinyl-, 5-Chlor-2-dichlormethyl-6-fluor-4-pyrimidinyl-, 5-Chlor-2-trichlormethyl-6-fluor-4-pyrimidinyl-, 5-Chlor-2-fluor-dichlormethyl-6-fluor-4-pyrimidinyl-, 2,6-Difluor-5-brom-4-pyrimidinyl-, 2-Fluor-5-brom-6-methyl-4-pyrimidinyl-, 2-Fluor-5-brom-6-chlormethyl-4-pyrimidinyl-, 2,6-Difluor-5-chlormethyl-4-pyrimidinyl-, 2,6-Difluor-5-nitro-4-pyrimidinyl-, 2-Fluor-6-methyl-4-pyrimidinyl-, 2-Fluor-5-chlor-6-methyl-4-pyrimidinyl-, 2-Fluor-6-chlor-4-pyrimidinyl-, 6-Trifluormethyl-5-chlor-2-fluor-4-pyrimidinyl-, 6-Trifluormethyl-2-fluor-4-pyrimidinyl-, 2-Fluor-5-nitro-4-pyrimidinyl-, 2-Fluor-5-trifluormethyl-4-pyrimidinyl-, 2-Fluor-5-phenyl- oder -5-methylsulfonyl-4-pyrimidinyl-, 2-Fluor-5-carbonamido-4-pyrimidinyl-, 2-Fluor-5-brom-6-trifluormethyl-4-pyrimidinyl-, 2-Fluor-6-carbonamido-4-pyrimidinyl-, 2-Fluor-6-carbomethoxy-4-pyrimidinyl-, 2-Fluor-6-phenyl-4-pyrimidinyl-, 2-Fluor-6-cyan-4-pyrimidinyl-, 5-Chlor-6-fluor-2-Methyl-4-pyrimidinyl-, 5,6-Difluor-2-Trifluormethyl-4-pyrimidinyl-, 5-Chlor-6-fluor-2-dichlorfluormethyl-4-pyrimidinyl-, 2-Fluor-5-chlorpyrimidin-4-yl, 2-Methyl-4-fluor-5-methylsulfonylpyrimidinyl-6, 2,6-Difluor-5-methyl-sulfonyl-4-pyrimidinyl-, 2,6-Dichlor-5-methylsulfonyl-4-pyrimidinyl, 2-Fluor-5-sulfonamido-4-pyrimidinyl-, 2-Fluor-5-chlor-6-carbomethoxy-4-pyrimidinyl-, 2,6-Difluor-5-trifluormethyl-4-pyrimidinyl; sulfonylgruppenhaltige Triazinreste, wie 2,4-Bis(phenylsulfonyl)-triazinyl-6-, 2-(3'-Carboxyphenyl)-sulfonyl-4-chlortriazinyl-6-, 2-(3-Sulfophenyl)-sulfonyl -4-chl or-triazinyl-6-, 2,4-Bis-(3'-carboxyphenylsulfonyl)-triazinyl-6; sulfonylgruppenhaltige Pyrimidinringe, wie 2-Carboxymethylsulfonylpyrimindinyl-4-, 2-Methylsulfonyl-6-methyl-pyrimidinyl-4-, 2-Methylsulfonyl-6-ethyl-pyrimidinyl-4-, 2-Phenylsulfonyl-5-chlor-6-methyl-pyrimidinyl-4-, 2,6-Bis-methyl sulfonyl-pyrimidinyl-4-, 2,6-Bis-methylsulfonyl-5-chlor-pyrimidinyl-4-' 2,4-Bis-methylsulfonyl-pyrimidin-5-sulfonyl-, 2-Methylsulfonyl-pyrimidinyl-4-, 2-Phenylsulfonyl-pyrimidinyl-4-, 2-Trichlormethylsulfonyl-6-methyl-pyrimidinyl-4-, 2-Methylsulfonyl-5-chlor-6-methyl-pyrimidinyl-4-, 2-Methylsulfonyl-5-brom-6-methyl-pyrimidinyl-4-, 2-Methylsulfonyl-5-chlor-6-ethyl-pyrimidinyl-4-, 2-Methylsulfonyl-5-chlor-6-chlormethyl-pyrimidinyl-4-, 2-Methylsulfonyl-4-chlor-6-methylpyrimidin-5-sulfonyl-, 2-Methylsulfonyl-5-nitro-6-methylpyrimidinyl-4-, 2,5,6-Tris-methylsulfonyl-pyrimidinyl-4-, 2-Methylsulfonyl-5,6-dimethyl-pyrimidinyl-4-, 2-Ethylsulfonyl-5-chlor-6-methyl-pyrimidinyl-4-, 2-Methylsulfonyl-6-chlor-pyrimidinyl-4-, 2,6-Bis-methylsulfonyl-5-chlor-pyrimidinyl-4-, 2-Methylsulfonyl-6-carboxypyrimidinyl-4-, 2-Methylsulfonyl-5-sulfo-pyrimidinyl-4-, 2-Methylsulfonyl-6-carbomethoxy-pyrimidinyl-4-, 2-Methylsulfonyl-5-carboxy-pyrimidinyl-4-, 2-Methylsulfonyl-5-cyan-6-methoxy-pyrimidinyl-4-, 2-Methylsulfonyl-5-chlorpyrimidinyl-4-, 2-β-Sulfoethylsulfonyl-6-methyl-pyrimidinyl-4-, 2-Methylsulfonyl-5-brom-pyrimidinyl-4-, 2-Phenylsulfonyl-5-chlor-pyrimidinyl-4-, 2-Methylsulfonyl-6-chlorpyrimidinyl-4- und -5-carbonyl-, 2,6-Bis-(methylsulfonyl)-pyrimidin-4 oder -5-carbonyl-, 2-Ethylsulfonyl-6-chlorpyrimidin-5-carbonyl-, 2,4-Bis(methylsulfonyl)-pyrimidin-5-sulfonyl-, 2-Methylsulfonyl-4-chlor-6-methylpyrimidin-5-sulfonyl- oder -carbonyl-; 2-Chlorbenzthiazol-5- oder -6-carbonyl- oder -5- oder -6-sulfonyl-, 2-Arylsulfonyl- oder Alkylsulfonylbenzthiazol-5- oder -6-carbonyl- oder -5- oder -6-sulfonyl-, wie 2-Methylsulfonyl- oder 2-Ethylsulfonylbenzthiazol-5- oder -6-sulfonyl- oder -carbonyl-, 2-Phenylsulfonylbenzthiazol-5- oder -6-sulfonyl- oder -carbonyl und die entsprechenden im ankondensierten Benzolring Sulfogruppen enthaltenden 2-Sulfonylbenzthiazol-5- oder -6-carbonyl- oder -sulfonyl-Derivate, 2-Chlorbenzoxazol-5- oder -6-carbonyl- oder -sulfonyl-, 2-Chlorbenzimidazol-5- oder -6-carbonyl- oder -sulfonyl-, 2-Chlor-1 -methylbenzimidazol-5- oder -6-carbonyl- oder -sulfonyl-, 2-Chlor-4-methylthiazol-(1,3)-5-carbonyl- oder -4- oder -5-sulfonyl-, N-Oxid des 4-Chlor- oder 4-Nitrochinolin-5-carbonyl.

Bei X = Pyrimidin sind insbesondere solche der Formel
X = 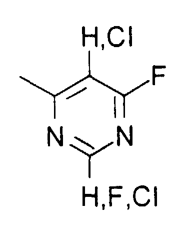 zu nennen.

Ganz besonders bevorzugt sind Farbstoffe der Formel (1), worin
X = 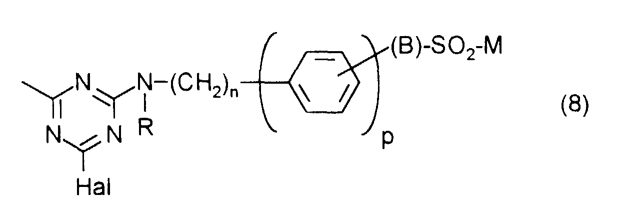 mit n = 0-3; p = 0-1; B = -(CH₂)ₘ- (CH₂)₂-O-(CH₂)₂; m = 0-6; Hal = Cl, F und R und M die obige Bedeutung besitzen, wobei insbesondere die folgenden zu nennen sind
- Hal =: Cl, F
- R =: Phenyl; n = 0; p = 0; m = 2 oder
- R =: H; n = 0; p = 1; m = 1, wobei der Phenylring zwischen 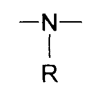 und -CH₂-SO₂M meta substituiert ist,
- R =: H; n = 0; p = 0; m = 3 oder
- R =: H; n = 0; p = 1 (para); m = 0, wobei der Phenylenring zwischen 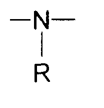 und -SO₂M para substituiert ist,
- R =: H; n = 0; B = -(CH₂)₂-O-(CH₂)₂-.

Weiterhin ganz besonders bevorzugt sind Farbstoffe der Formel (1), worin
X = 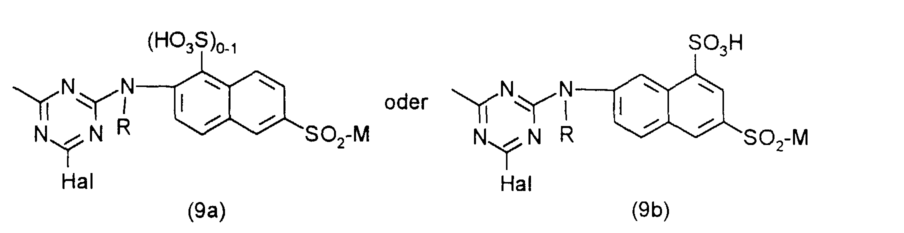 mit vorzugsweise
- Hal =: Cl, F,
- R =: H,
- M =: hat die unter Formel (1) angegebene Bedeutung.

Bevorzugt als Diazokomponenten sind die in DE-A-2 208 972, EP-A-0 088 930 und EP-A-0 013 879 beschriebenen Triazole. Diese können als reaktive Diazokomponenten eingesetzt werden oder auch erst nach der Kupplung mit Reaktivanker umgesetzt werden.

Die Erfindung betrifft weiterhin ein Verfahren zur Herstellung der Farbstoffe (1), dadurch gekennzeichnet, daß man Verbindungen der Formel (10) 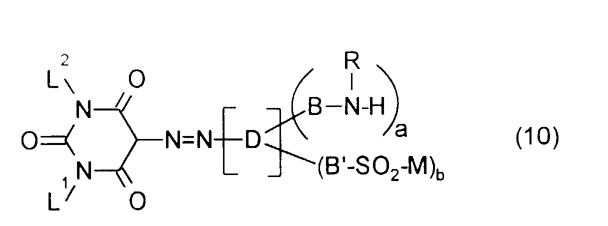 bzw. die entsprechenden Diazokomponenten der Formel 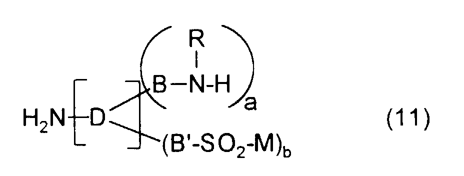 wobei a = 0, 1 oder 2; b = 0, 1 oder 2 und a + b = 1 oder 2 bedeuten und die übrigen Substituenten die obigen Bedeutungen besitzen,
mit 1 bis 2 Mol-Äquivalenten einer Reaktivkomponente der Formel X-Hal umsetzt, wobei X einen faserreaktiven heterocyclischen Rest bedeutet und Hal für Halogen steht, insbesondere F und Cl, und im Falle der Verwendung von Vorprodukten diese anschließend in die gewünschten Endfarbstoffe überführt und gegebenenfalls weitere Umwandlungsreaktionen anschließt.

Bei der Herstellung der bevorzugten Azofarbstoffe muß die Diazokomponente zwei Aminogruppen oder eine Aminogruppe und einen Reaktivrest -SO₂M bzw. eine Vorstufe des Restes -SO₂M enthalten. Die Diazokomponente kann gegebenenfalls weitere acylierbare Aminogruppen enthalten. Gegebenenfalls verwendet man entsprechende Acylamino- oder Nitroverbindungen, worin die Acylamino- bzw. Nitrogruppe vor der Kondensation mit einem Halogentriazin, Halogenpyrimidin oder dergleichen durch Verseifen bzw. Reduzieren in die NH₂-Gruppe übergeführt wird. Die Einführung des Reaktivrests X erfolgt durch Kondensation von Farbstoffen oder Farbstoffvorprodukten, welche acylierbare Aminogruppen enthalten, mit faserreaktiven halogenierten Acylierungsmitteln. Bei der Herstellung der Endfarbstoffe aus Vorprodukten handelt es sich meistens um Kupplungen, die zu Azofarbstoffen führen.

Da die einzelnen oben angegebenen Verfahrensschritte in unterschiedlicher Reihenfolge ausgeführt werden können, sind verschiedene Verfahrensvarianten möglich. Im allgemeinen führt man die Umsetzung schrittweise nacheinander aus, wobei die Reihenfolge der einfachen Reaktionen zwischen den einzelnen Reaktionskomponenten vorteilhafterweise sich nach den besonderen Bedingungen richtet. Da unter bestimmten Voraussetzungen Hydrolyse des reaktiven Rests eintritt, muß ein Zwischenprodukt, welches Acylaminogruppen enthält, zwecks Abspaltung der Acylgruppen verseift werden, bevor mit einem Aminodifluortriazin oder Trifluortriazin etc. kondensiert wird. Als weitere Umwandlungsreaktion kommt z.B. die nachträgliche Umsetzung eines Dihalogentriazinylrestes mit einem Amin in Betracht. Die wichtigsten Verfahrensvarianten sind in den Ausführungsbeispielen dargestellt.

Geeignete Ausgangsverbindungen für die Herstellung von Mono- oder Polyazofarbstoffen (1) sind beispielsweise:

### Diazokomponenten

1,3-Diaminobenzol, 1,4-Diaminobenzol, 1,3-Diamino-4-chlorbenzol, 1,3-Diamino-4-methylbenzol, 1,3-Diamino-4-ethylbenzol, 1,3-Diamino-4-methoxybenzol, 1,3-Diamino-4-ethoxybenzol, 1,4-Diamino-2-methylbenzol, 1,4-Diamino-2-methoxybenzol, 1,4-Diamino-2-ethoxybenzol, 1,4-Diamino-2-chlorbenzol, 1,4-Diamino-2,5-dimethylbenzol, 1,4-Diamino-2,5-diethylbenzol, 1,4-Diamino-2-methyl-5-methoxybenzol, 1,4-Diamino-2,5-dimethoxybenzol, 1,4-Diamino-2,5-diethoxybenzol, 2,6-Diamino-naphthalin, 1,3-Diamino-2,4,6-trimethylbenzol, 1,4-Diamino-2,3,5,6-tetramethylbenzol, 1,3-Diamino-4-nitrobenzol, 4,4'-Diaminostilben, 4,4'-Diaminodiphenylmethan, 4,4'-Diaminobiphenyl (Benzidin), 3,3'-Dimethylbenzidin, 3,3'-Dimethoxybenzidin, 3,3'-Dichlorbenzidin, 3,3'-Dicarboxybenzidin, 3,3'-Dicarboxymethoxy-benzidin, 2,2'-Dimethylbenzidin, 4,2'-Diaminodiphenyl (Diphenylin), 2,6-Diaminonaphthalin-4,8-disulfonsäure, 1,4-Diaminobenzol-2-sulfonsäure, 1,4-Diaminobenzol-2,5-disulfonsäure, 1,4-Diaminobenzol-2,6-disulfonsäure, 1,3-Diaminobenzol-4-sulfonsäure, 1,3-Diaminobenzol-4,6-disulfonsäure, 1,4-Diamino-2-chlorbenzol-5-sulfonsäure, 1,4-Diamino-2-methylbenzol-5-sulfonsäure, 1,5-Diamino-6-methylbenzol-3-sulfonsäure, 1,3-Diamino-6-methylbenzol-4-sulfonsäure, 3-(3'-bzw. 4'-Aminobenzoylamino)-1-aminobenzol-6-sulfonsäure, 1-(4'-Aminobenzoylamino)-4-aminobenzol-2,5-disulfonsäure, 1,4-Diaminobenzol-2-carbonsäure, 1,3-Diaminobenzol-4-carbonsäure, 1,2-Diaminobenzol-4-carbonsäure, 1,3-Diaminobenzol-5-carbonsäure, 1,4-Diaminobenzol-2-methylbenzol, 4,4'-Diaminodiphenyloxid, 4,4'-Diaminodiphenylharnstoff-2,2'-disulfonsäure, 4,4'-Diaminodiphenyloxyethan-2,2'-disulfonsäure, 4,4'-Diaminostilben-2,2'-disulfonsäure, 4,4'-Diaminodiphenylethan-2,2'-disulfonsäure, 2-Amino-5-aminomethylnaphthalin-1-sulfonsäure, 2-Amino-5-aminomethylnaphthalin-1,7-disulfonsäure, 1-Amino-4-methoxy-5-aminomethylbenzol-6-sulfonsäure, l-Amino-4-methyl-5-aminomethylbenzol-6-sulfonsäure.

Wenn als Diazokomponente statt eines Diamins eine amino-acetylamino-Verbindung eingesetzt werden soll, aus welcher nachträglich die Acylgruppe durch Verseifen wieder abgespalten wird, wie dies oben in den Erläuterungen der Verfahrensvarianten beschrieben ist, kommen die Monoacylverbindungen der obengenannten Diazokomponenten in Frage, z.B. 1-Acetylamino-3-aminobenzol-4-sulfonsäure oder 1-Acetylamino-4-aminobenzol-3-sulfonsäure.

Die Diazotierung der Diazokomponenten bzw. der eine diazotierbare Aminogruppe enthaltenden Zwischenprodukte erfolgt in der Regel durch Einwirkung salpetriger Säure in wäßrig-mineralsaurer Lösung bei tiefer Temperatur. Die Kupplung auf die Kupplungskomponente erfolgt bei stark sauren, neutralen bis schwach alkalischen pH-Werten.

Kupplungskomponenten der Formel 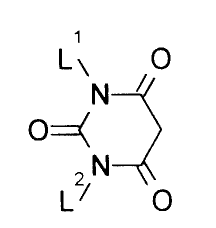 sind z.T. bekannt aus P.L. Southwick, A.S. Wagman und A.S. Waggoner, Org. Prep. Proced. Int. 23 (1991) 6, 713; P.L. Southwick und A.S. Waggoner, Org. Prep. Proced. Int. 21 (1989)4, 493; DOS 1 925 559 und DOS 2 711 450 bzw lassen sich analog herstellen.

Die Kondensation der Reaktivkomponenten mit den Diazokomponenten und mit den Aminen bzw. mit acylierbaren Monoazo- oder Disazo-Zwischenprodukten bzw. mit den aminogruppenhaltigen Farbstoffen erfolgen vorzugsweise in wäßriger Lösung oder Suspension, bei niedriger Temperatur und bei schwach saurem, neutralem bis schwach alkalischem pH-Wert. Vorteilhaft wird der bei der Kondensation freiwerdende Halogenwasserstoff laufend durch Zugabe wäßriger Alkalihydroxide, -carbonat oder -bicarbonate neutralisiert.

Die angegebenen Formeln sind die der freien Säuren. Bei der Herstellung werden im allgemeinen die Salze erhalten, insbesondere die Alkalisalze wie Natrium-, Kalium-oder Lithiumsalze. Die durch Quarternierung mit Pyridinen entstehende Ladung wird, in Abhängigkeit der Isolierungsbedingungen, durch ein Gegenion z.B. Chlorid, Fluorid oder Sulfat ausgeglichen; oder die Farbstoffe bilden innere Salze mit Sulfo- oder Carboxylgruppen.

Alle Farbstoffe, insbesondere solche die in letzter Stufe mit Pyridinen umgesetzt werden, können je nach Reaktionsbedingungen als Gemische der β-Sulfatoethylsulfonylfarbstoffe und deren eliminierten Form mit dem Vinylsulfon vorliegen. Die Farbstoffe können auch als konzentrierte Lösungen eingesetzt werden.

Die erfindungsgemäßen Farbstoffe eignen sich hervorragend zum Färben und Bedrucken von natürlichen und synthetischen OH- oder amidgruppenhaltigen Materialien, insbesondere solchen aus Cellulose und Polyamiden. Sie sind besonders geeignet zum Färben von Cellulosematerialien in Auszieh- und Klotz-Kaltverweilverfahren, sowie zum Bedrucken von Baumwolle und Zellwolle.

Man erhält bei gutem Aufbauvermögen und hohen Fixierausbeuten Färbungen mit guten Allgemeinechtheiten, insbesondere Naßechtheiten.

Ebenfalls Gegenstand der vorliegenden Erfindung sind Verbindungen der Formel (12) 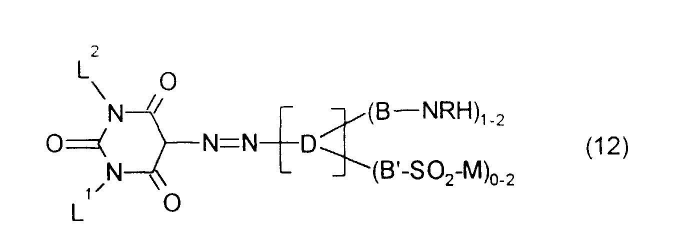 wobei L¹, L², B, B', R, D und M die obengenanten Bedeutungen haben.

Es handelt sich hierbei um wertvolle Zwischenprodukte zur Herstellung der reaktiven Farbstoffe der Formel (1).

Bevorzugte Verbindungen der Formel (12) sind solche, deren Substituenten die obigen bevorzugten Bedeutungen besitzen.

### Färbevorschriften

Die in den folgenden Beispielen angegebenen Färbungen werden unter folgenden Bedingungen durchgeführt:

### Färbevorschrift 1

2 Teile des Farbstoffes werden in 100 ml Wasser gelöst. Die Lösung gibt man zu 1.900 Teilen kaltem Wasser, fügt 60 Teile Natriumchlorid zu und geht mit 100 Teilen eines Baumwollgewebes in dieses Färbebad ein.

Man steigert die Temperatur auf 60°C, wobei nach 30 Minuten 40 Teile kalziniertes Soda und nochmals 60 Teile Natriumchlorid zugegeben werden. Man erhält die Temperatur 30 Minuten auf 60°C, spült und seift dann die Färbung während 15 Minuten in einer 0,3 %igen kochenden Lösung eines ionenfreien Waschmittels, spült und trocknet.

### Färbevorschrift 2

4 Teile des Reaktivfarbstoffes werden in 50 Teilen Wasser gelöst. Dazu gibt man 50 Teile einer Lösung, die pro Liter 5 g Natriumhydroxid und 10 g kalziniertes Soda enthält. Mit der erhaltenen Lösung wird ein Baumwollgewebe foulardiert, so daß es um 70 % seines Gewichtes zunimmt, und dann auf eine Kaule aufgewickelt. Das Baumwollgewebe wird so während 3 bis 12 h bei Raumtemperatur gelagert. Danach wird die gefärbte Ware gespült, während einer Viertelstunde mit einem nichtionogenen Waschmittel kochend geseift, nochmals gespült und getrocknet.

### Beispiel 1

0,29 Mol 4-(2-Sulfatoethyl)sulfanylanilin werden in bekannter Weise diazotiert und auf 0,29 Mol 1-(2-Sulfoethyl)barbitursäure oder das Mono- bzw. Di-Natriumsalz bei pH 5-7 gekuppelt.

Der nach Aussalzen, Isolierung, Trocknung und Mahlen erhaltene Farbstoff der Formel 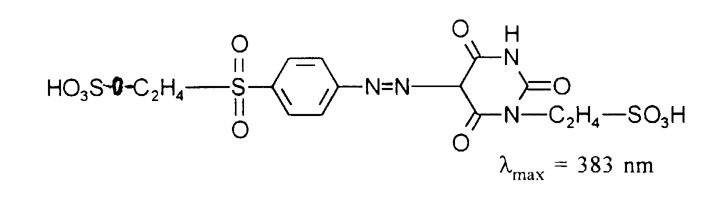 färbt Baumwolle in grünstichig gelben Tönen.

Durch Variation der Diazokomponenten und Kupplungskomponenten werden weitere wertvolle grünstichig gelbe Reaktivfarbstoffe (Beispiele 2 bis 6) erhalten.

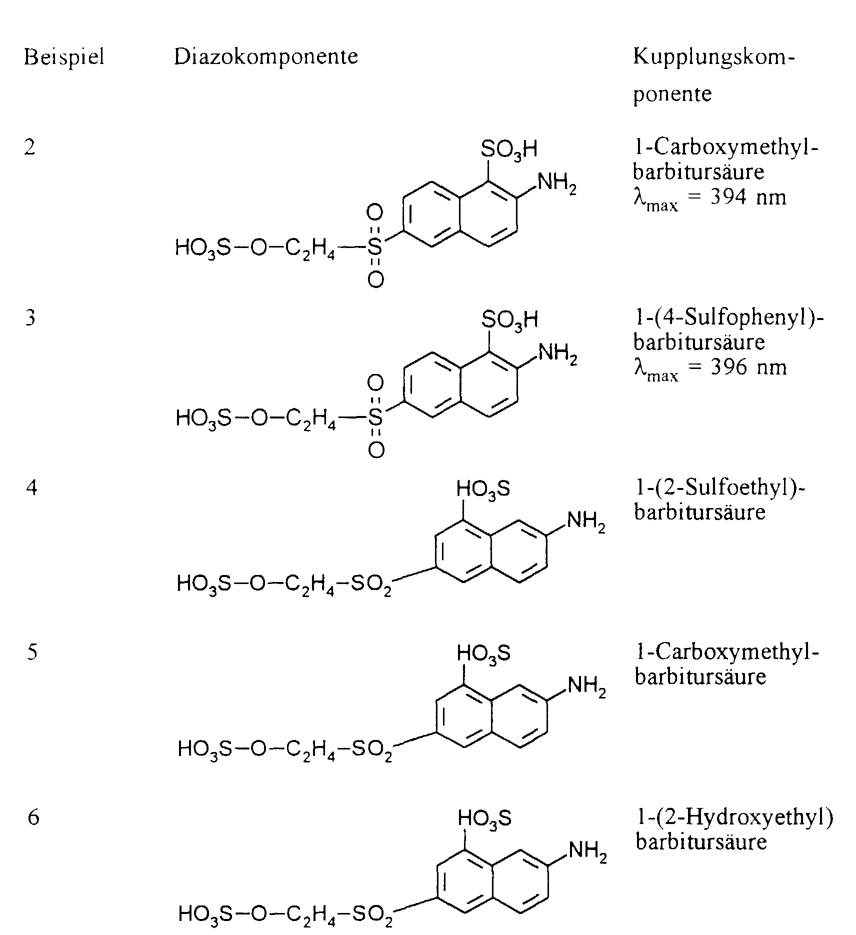

### Beispiel 7

0,29 Mol 4-Acetamino-2-amino-benzolsulfonsäure werden in bekannter Weise diazotiert und auf 0,29 Mol 1-(2-Sulfoethyl)barbitursäure bei pH 5-7 gekuppelt. Anschließend wird in bekannter Weise verseift. Der nach Aussalzen und Isolierung erhaltene Farbstoff der Formel 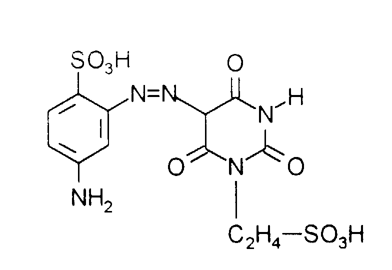 läßt sich mit beliebigen heterocyclischen Reaktivhaken umsetzen.

0,2 Mol des obigen Farbstoffs werden in 600 ml Wasser bei pH 7,0 gelöst und mit 600 g Eis auf 0°C abgekühlt. 0,21 Mol 2,4,6-Trifluor-1,3,5-triazin (Cyanurfluorid) werden zugegeben und der pH mit Na₂CO₃-Lösung zwischen 3,5 und 4 gehalten. Nach 5 min setzt man 0,2 Mol Morpholin zu und stellt den pH mit Na₂CO₃-Lösung auf 7 ein. Die Temperatur steigt dabei auf ca. 10°C an. Es wird 60 min bei Raumtemperatur nachgerührt. Anschließend wird der Farbstoff ausgesalzen, isoliert, getrocknet und gemahlen. Der so erhaltene Farbstoff der Formel 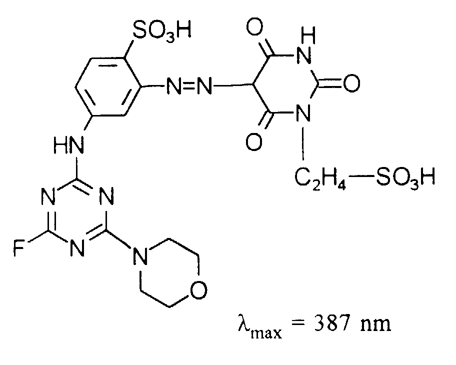 färbt Baumwolle in klaren grünstichig gelben Tönen.

Durch Variation der Diazokomponenten (D), Kupplungskomponenten (K) und Reaktivhaken erhält man weitere wertvolle grünstichig gelbe Reaktivfarbstoffe der Formel

K-N=N-D-X.

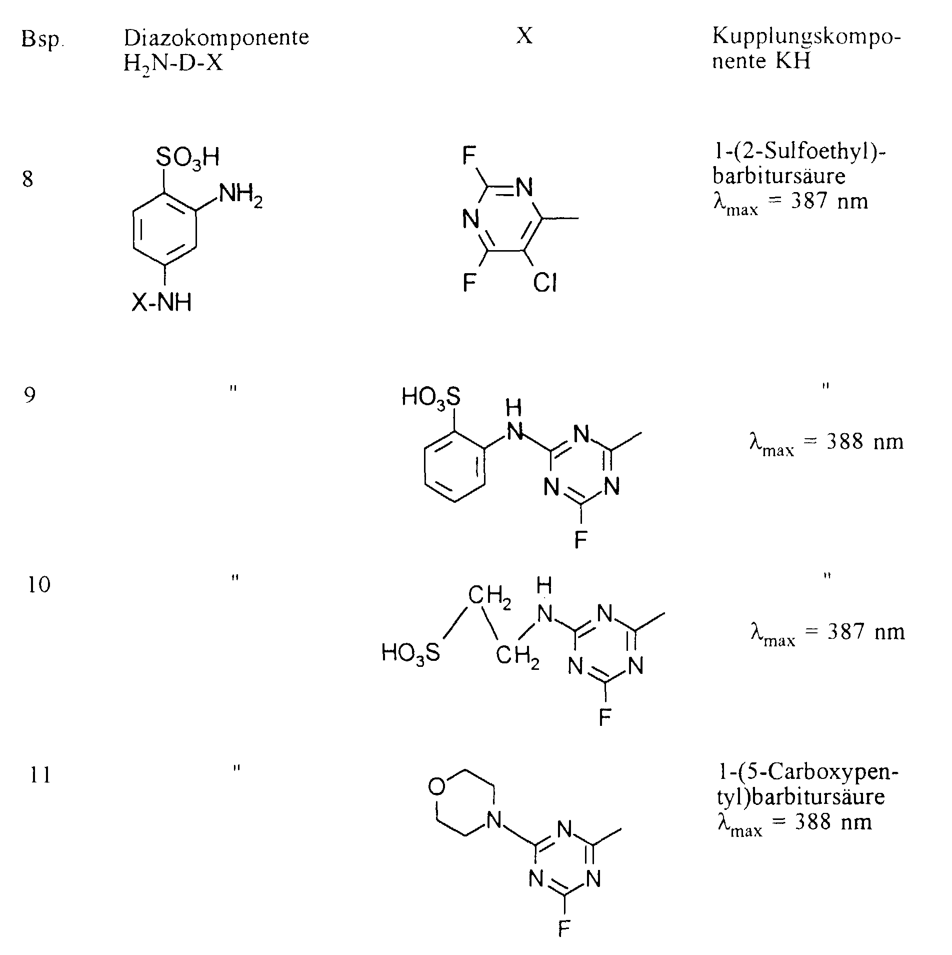

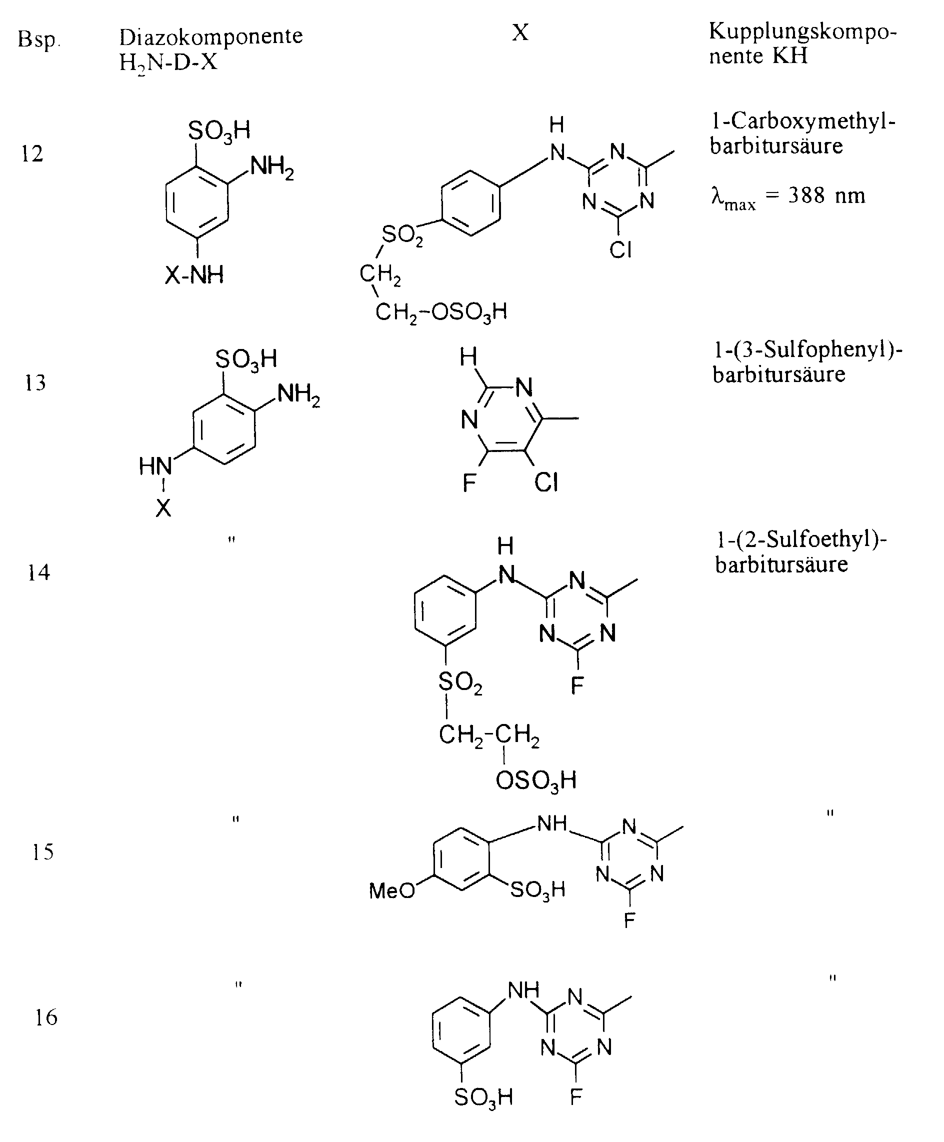

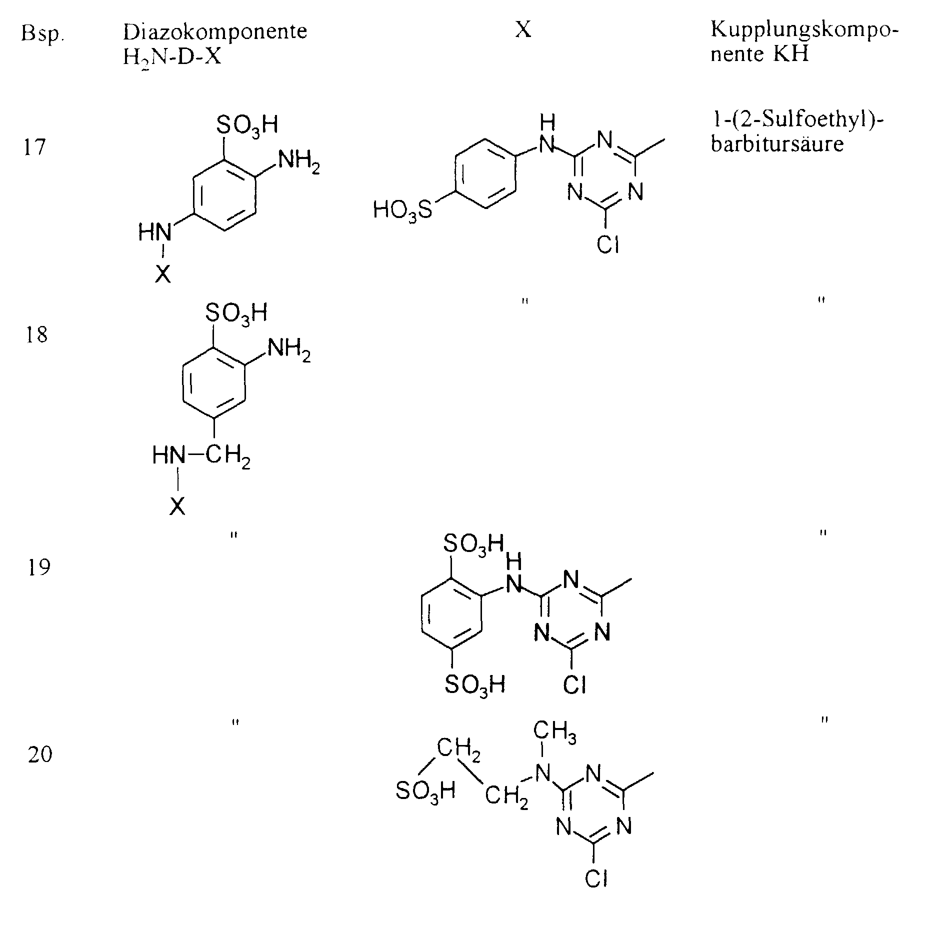

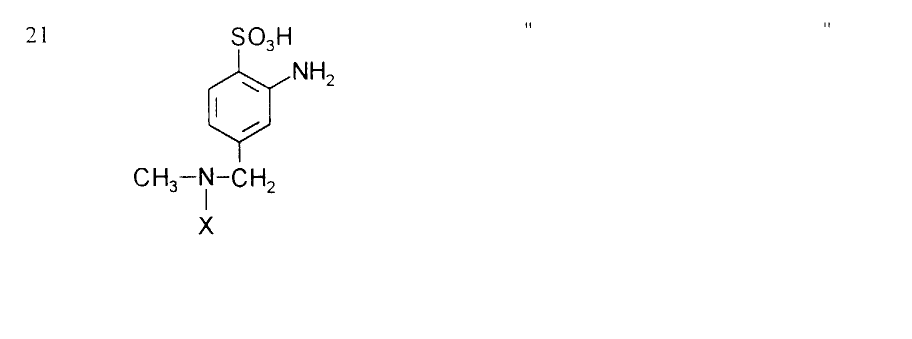

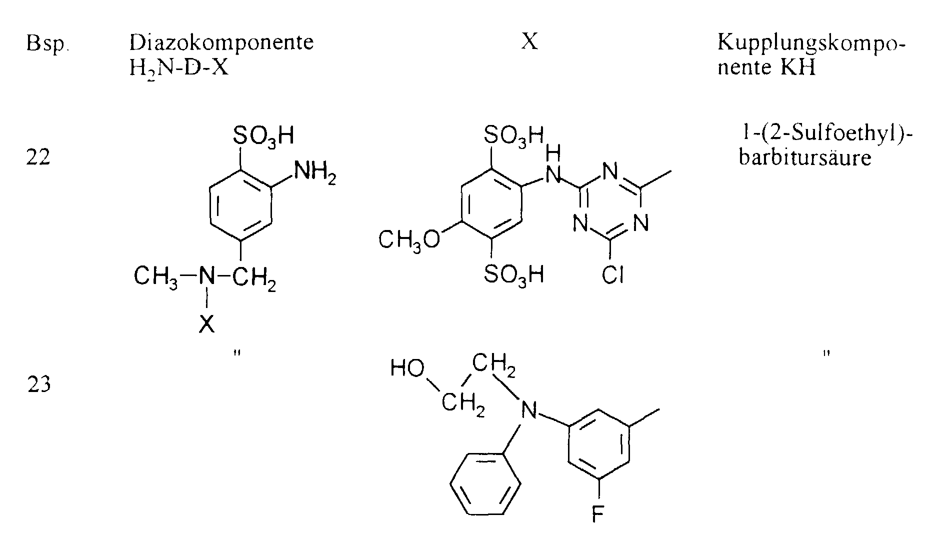

### Beispiel 25

0,29 Mol 2-Amino-5-aminomethyl-1-naphthalinsulfonsäure werden in bekannter Weise diazotiert und auf 0,3 Mol 1-(2-Sulfoethyl)barbitursäure gekuppelt. Eine Lösung von 0,32 Mol 2-(2',5'-Disulfophenyl-amino)-4,6-dichlor-1,3,5-triazin-Di-Na-Salz in 400 ml Wasser wird zur Kupplung gegeben. Anschließend wird 2 Stunden auf 60°C erwärmt. Der pH-Wert wird mit Sodalösung bei pH = 6-7 gehalten.

Der nach Aussalzen, Isolierung, Trocknung und Mahlen erhaltene Farbstoff der Formel 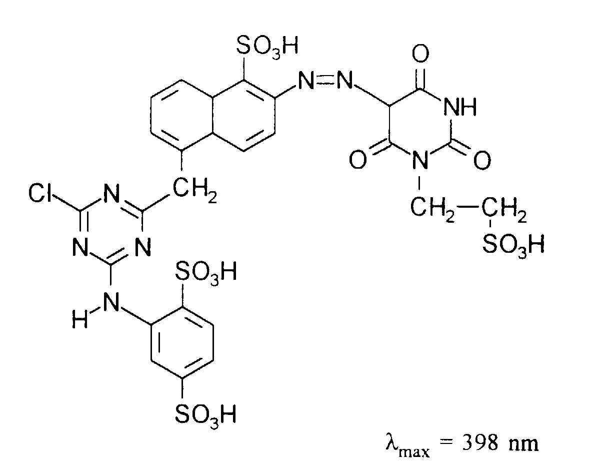 färbt Baumwolle in grünstichig gelben Tönen.

Weitere grünstichig gelbe Reaktivfarbstoffe K-N=N-D-X erhält man durch Umsetzung der folgenden Komponenten.

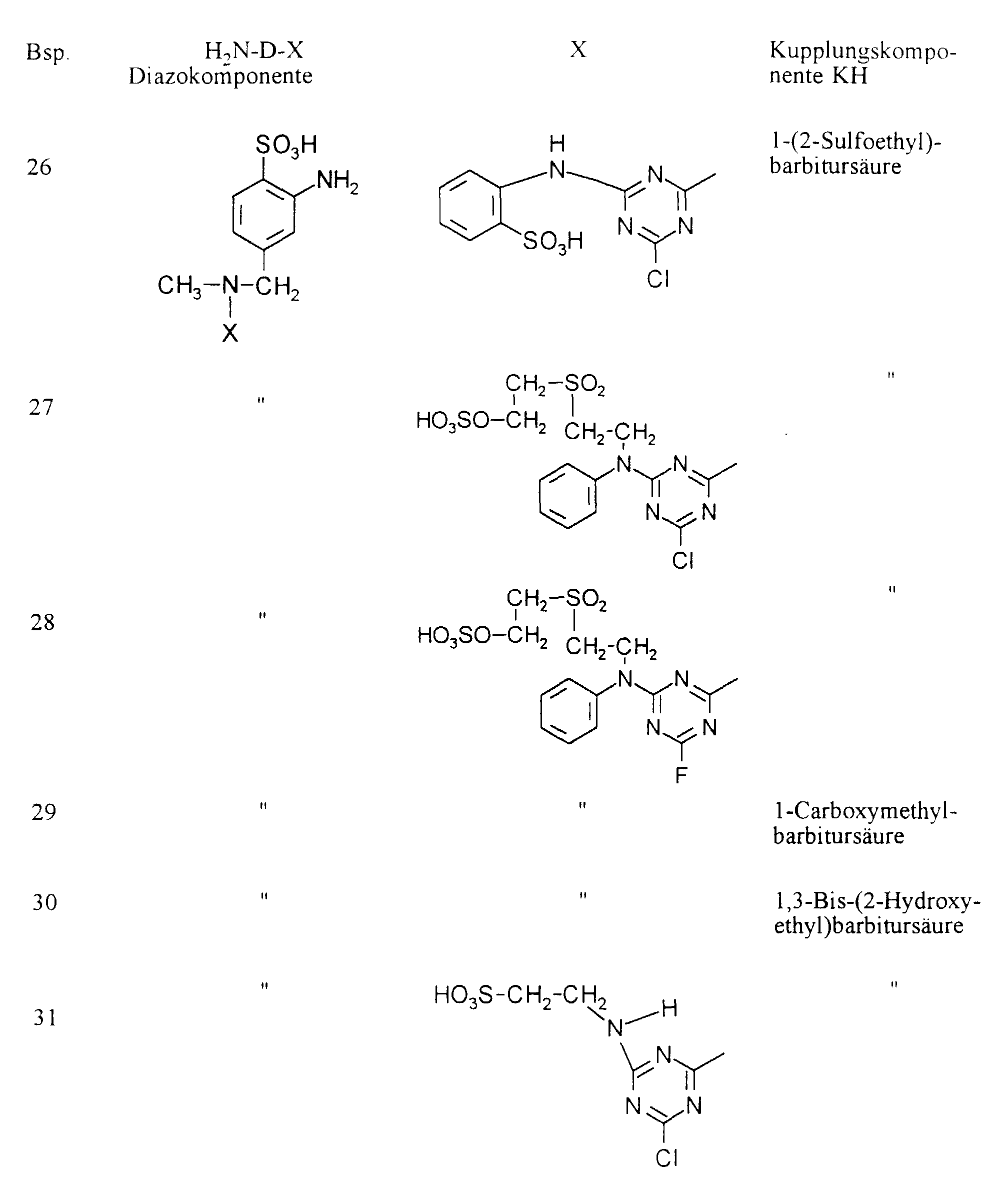

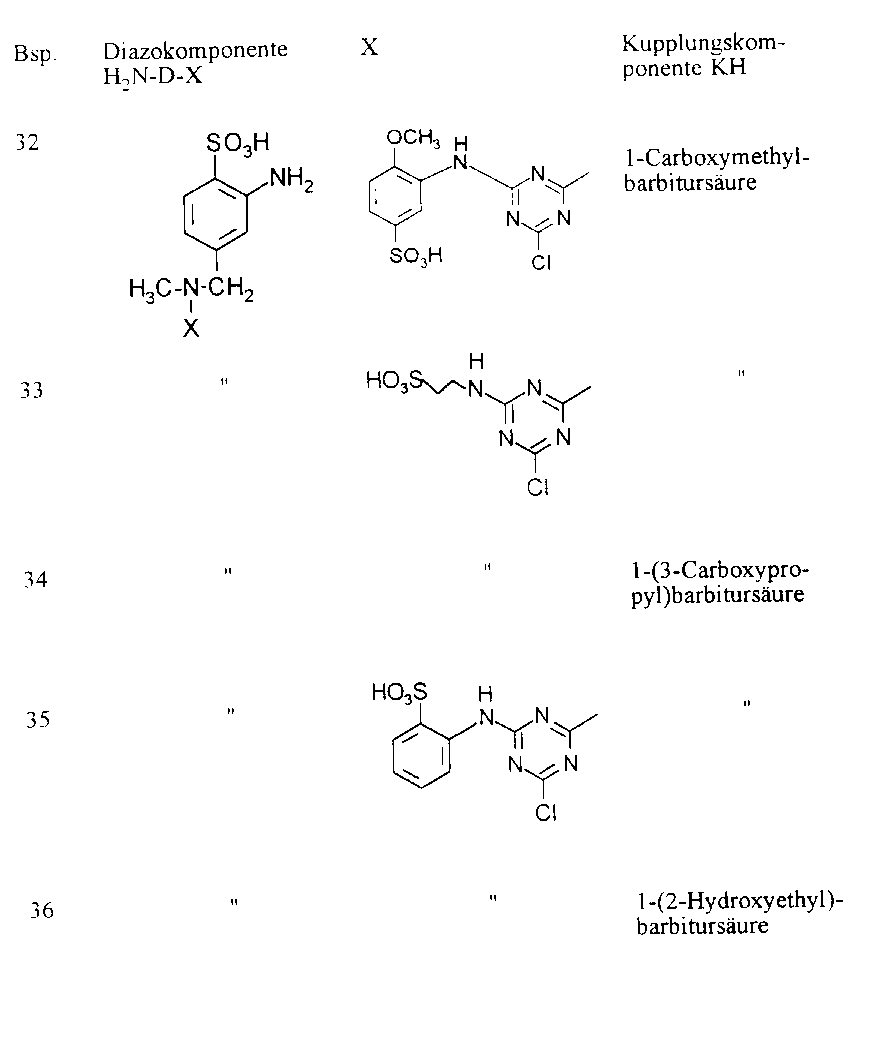

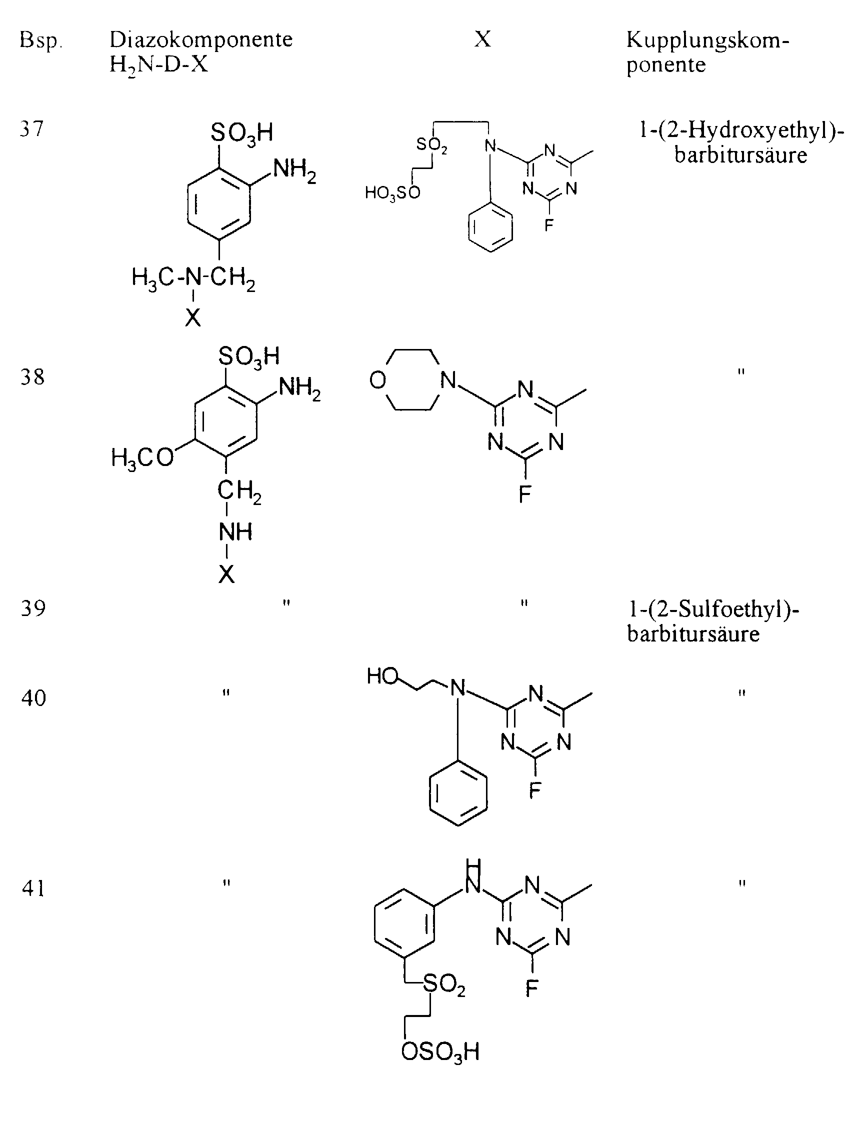

### Beispiel 42

0,29 Mol 4-(2-Sulfatoethyl)sulfonylanilin werden in bekannter Weise diazotiert und zu einer neutralen, auf 15°C abgekühlten Lösung von 0,29 Mol 4-(4'-Amino-2'-sulfophenyl)-amino-5-chlor-2,6-difluorpyridin in 2 l Wasser gegeben. Während der Kupplung wird ein pH-Wert von 5,5 mit Sodalösung gehalten. Die wässrige Kupplungslösung wird auf 0-5°C abgekühlt. Bei pH = 2,5 bis 2,8 werden 70 ml 30%ige Natriumnitritlösung zugegeben. Die Diazotierung rührt 1 Stunde bei 0 bis 5°C nach. Anschließend wird überschüssiges Nitrit durch Zugabe von Amidosulfonsäure zerstört.

0,32 Mol 1-(2-Sulfoethyl)barbitursäure werden eingestreut. Mit Sodalösung wird ein pH-Wert von 6,5 eingestellt. Nach Beendigung der Kupplung wird der Farbstoff durch Ethanol ausgefällt, abgesaugt und getrocknet. Der Farbstoff hat die Formel 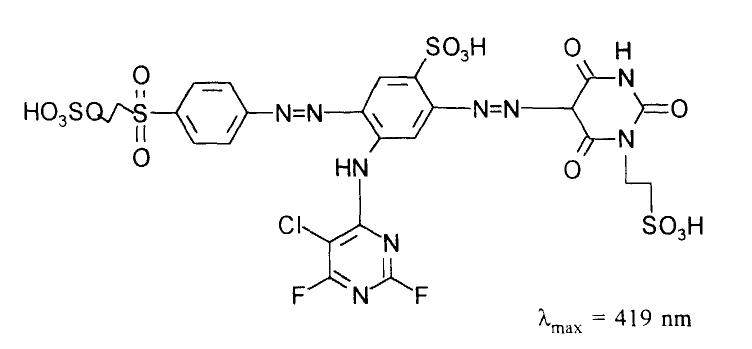 und färbt Baumwolle in rotstichig gelben Tönen. Weitere ähnliche Cellulosefasern rotstichig gelb färbende Reaktivfarbstoffe K-N=N-D-X werden erhalten, wenn man die in nachfolgender Zusammenstellung angegebenen Komponenten miteinander umsetzt.

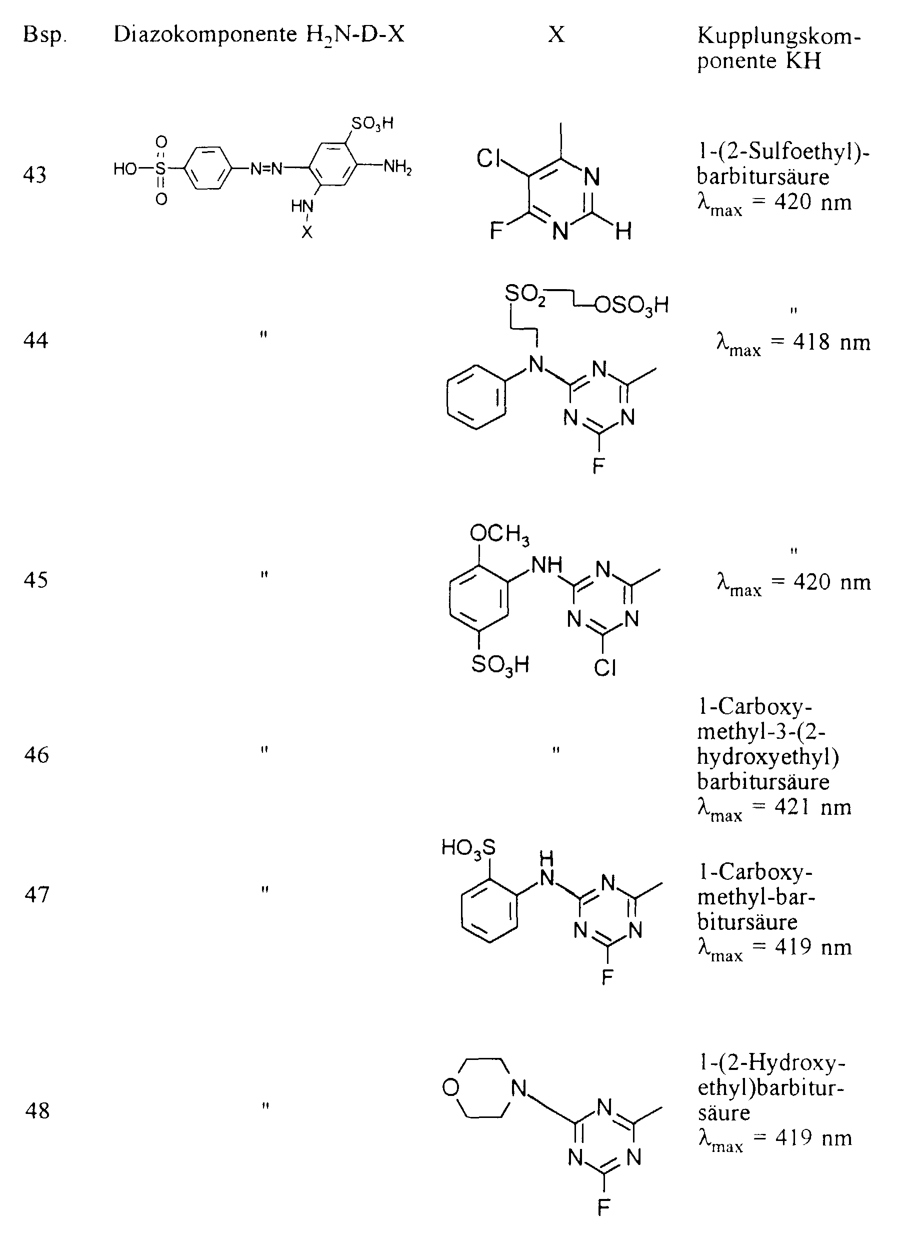

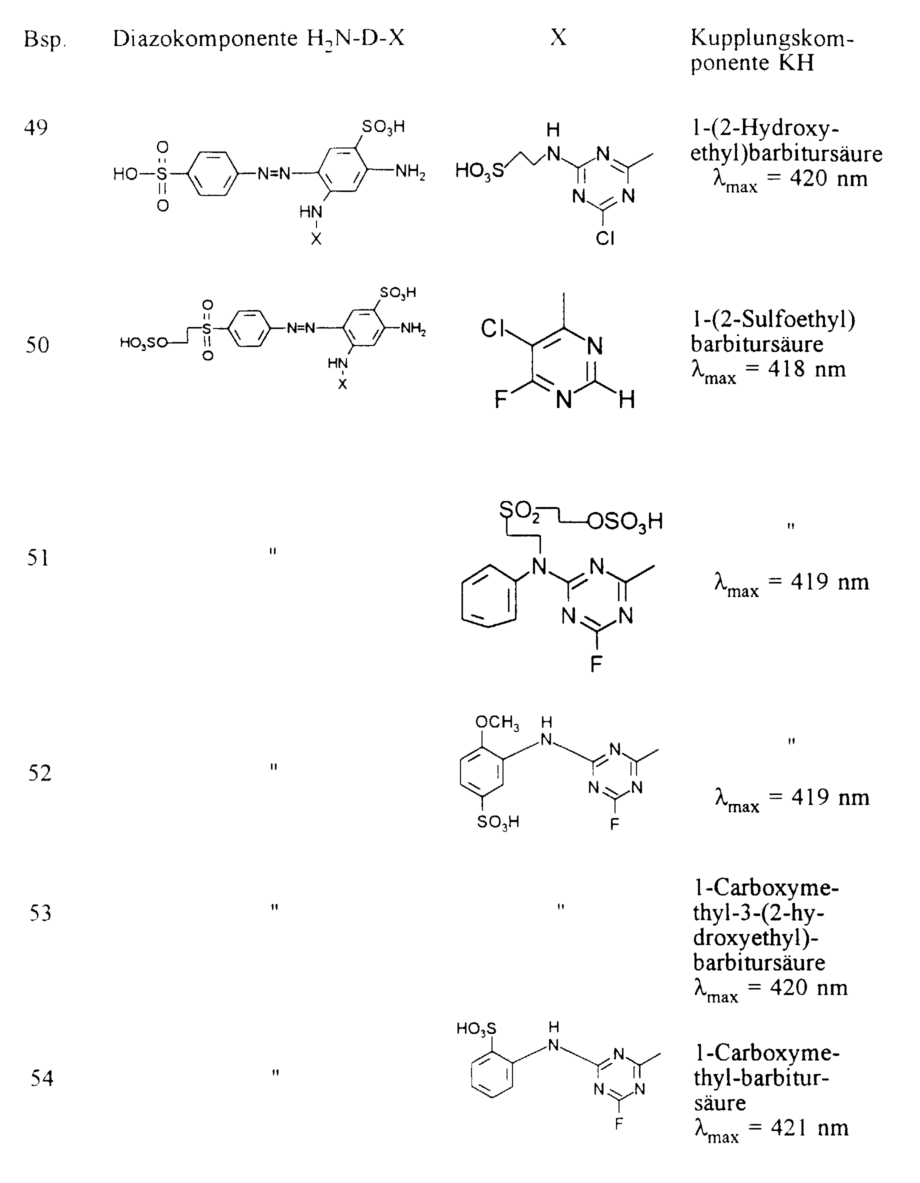

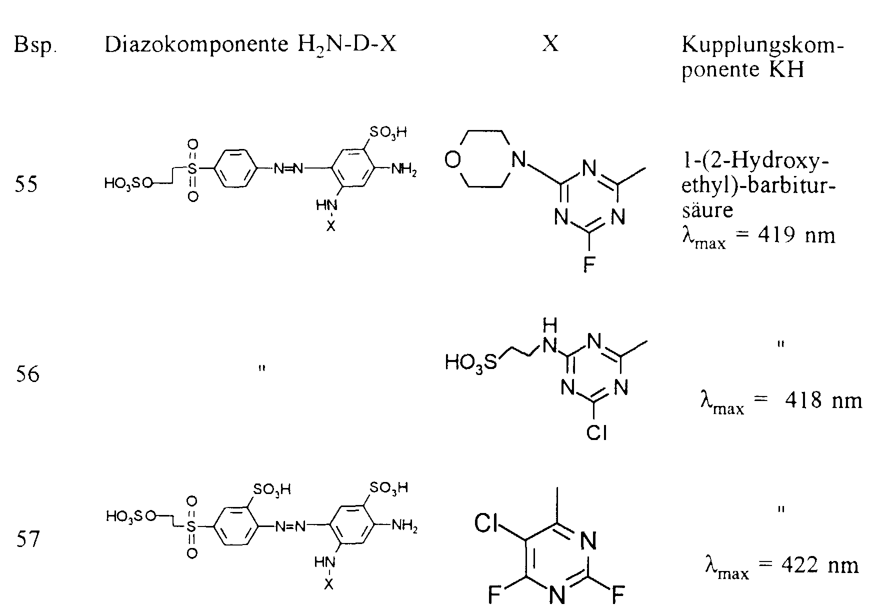

### Beispiel 58

0,29 Mol 4-(2-Sulfatoethyl)sulfonylanilin werden in bekannter Weise diazotiert. 0,29 Mol m-Toluidin werden in 30 ml Eisessig gelöst. Die Lösung wird langsam zur Diazotierung getropft. Anschließend wird 220 ml 20%ige Natriumacetatlösung auf einen pH-Wert von ca. 4,0 eingestellt. Der ausgefallene orange Farbstoff wird abfiltriert, getrocknet und gemahlen. Das Farbstoffpulver wird in 400 ml 20%iges Oleum eingetragen. Die Temperatur steigt auf 80°C. Die Lösung wird 2 Stunden bei 80°C und 3 Stunden bei 60°C gerührt. Nach Abkühlen auf Raumtemperatur wird die Lösung auf Eis/Kaliumchloridsole gegeben. Der Farbstoff wird abgesaugt und in 1 l Wasser verrührt. Mit 70 ml 30%iger Natriumnitritlösung wird 1 Stunde bei 20°C nachgerührt. Der Nitritüberschuß wird mit Amidosulfonsäure vernichtet. 0,29 Mol 1-(2-Sulfoethyl)barbitursäure werden eingestreut. Der pH-Wert wird mit Sodalösung auf 6,5 gestellt. Es wird 1 Stunde bei 20°C nachgerührt. Der nach Aussalzen, Isolierung, Trocknung und Mahlen erhaltene Farbstoff der Formel 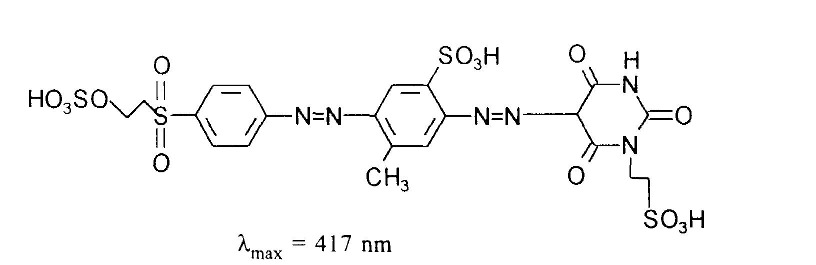 färbt Baumwolle in rotstichig gelben Tönen. Weitere analog herstellbare Reaktivfarbstoffe K-N=N-D-SO₂-M sind nachstehend angegeben:

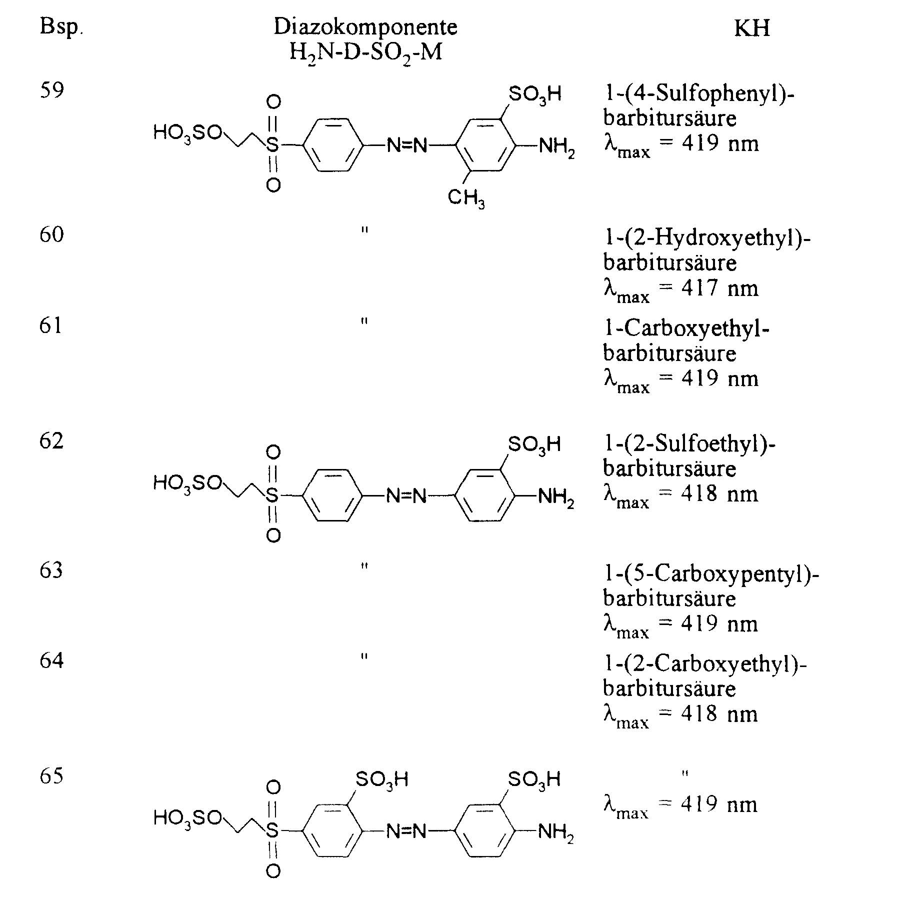

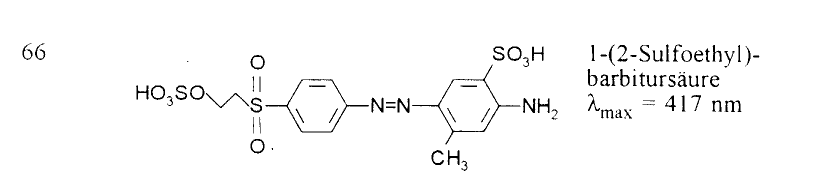

### Beispiel 67

Eine Lösung von 0,348 Mol l 1-Aminobenzolsulfonsäure in 1 Wasser wird bei 0°C und pH = 4 bis 4,5 mit 0,39 Mol 2,4,6-Trifluor-1,3,5-triazin in 5 bis 10 Minuten versetzt. Es wird 10 Minuten nachgerührt. Zu einer neutralen Lösung von 0,29 Mol des Triazols der Formel 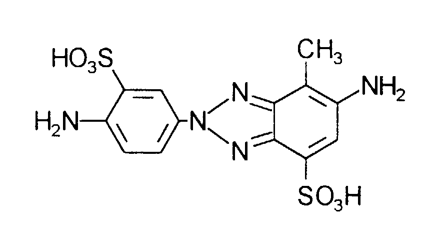 in 2,5 l Wasser werden 120 g Natriumhydrogencarbonat gegeben. Nach Abkühlen der Lösung auf 15°C wird obige Mischung in 10 bis 15 Minuten zugegeben. Es wird 2 bis 3 Stunden bei Raumtemperatur nachgerührt. Nach Abkühlen der Mischung auf 0 bis 5°C und Zugabe von 70 ml 30%iger Natriumnitritlösung wird mit 30%iger Salzsäure ein pH-Wert von 2,8 bis 3,0 eingestellt.

Es wird 30 Minuten nachgerührt. Nach Zerstören des Natriumnitritüberschusses mit Amidosulfonsäure werden 0,3 Mol 1-(2'-Sulfoethyl)-barbitursäure in die Diazotierung gegeben. Mit Sodalösung wird ein pH-Wert von 6,5 eingestellt. Es wird 1 Stunde bei Raumtemperatur und pH = 6,5 nachgerührt. Der nach Aussalzen, Isolierung, Trocknung und Mahlen erhaltene Farbstoff der Formel 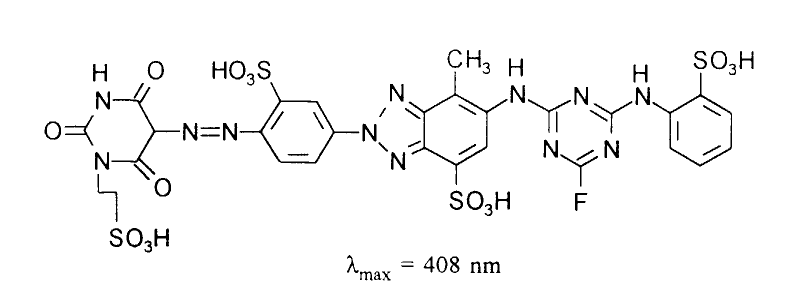 färbt Baumwolle in grünstichig gelben Tönen.

### Beispiel 68

Einer Lösung von 0,29 Mol des Triazols der Formel 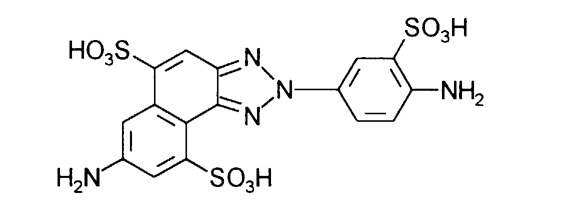 in 3,8 l Wasser werden bei 0°C und pH = 6,5 0,334 Mol 2,4,6-Trifluor-1,3,5-triazin in ca. 10 Minuten zugesetzt. Mit Natriumhydrogencarbonatlösung wird ein pH-Wert von 5,5 gehalten. Nach 10 Minuten werden 0,318 Mol N-(2-Hydroxyethyl)anilin zugegeben. Mit Sodalösung wird ein pH-Wert von 7,6 eingestellt. Es wird 2 Stunden bei Raumtemperatur nachgerührt. Anschließend wird bei 5 bis 10°C und pH = 2,8 diazotiert. Nach Zerstören des Nitritüberschusses werden 3 Mol 1-(2-Sulfoethyl)barbitursäure in die Diazotierung gegeben. Mit Sodalösung wird ein pH-Wert von 6,5 bis 7 eingestellt. Der nach Aussalzen, Isolierung, Trocknung und Mahlen erhaltene Farbstoff der Formel 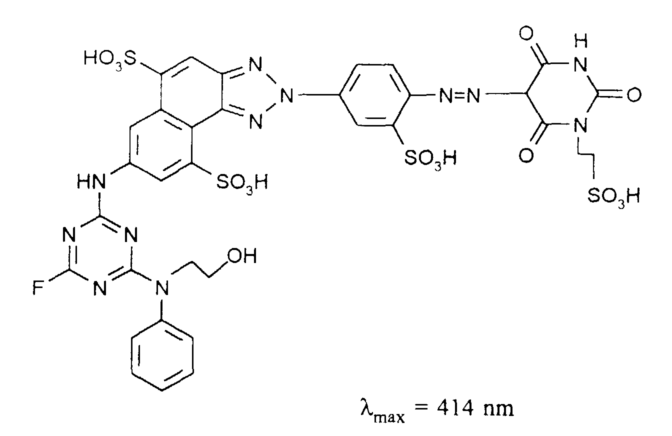 färbt Baumwolle in grünstichig gelben Tönen.

Weitere wertvolle Reaktivfarbstoffe dieses Typs K-N=N-D-X lassen sich analog herstellen.

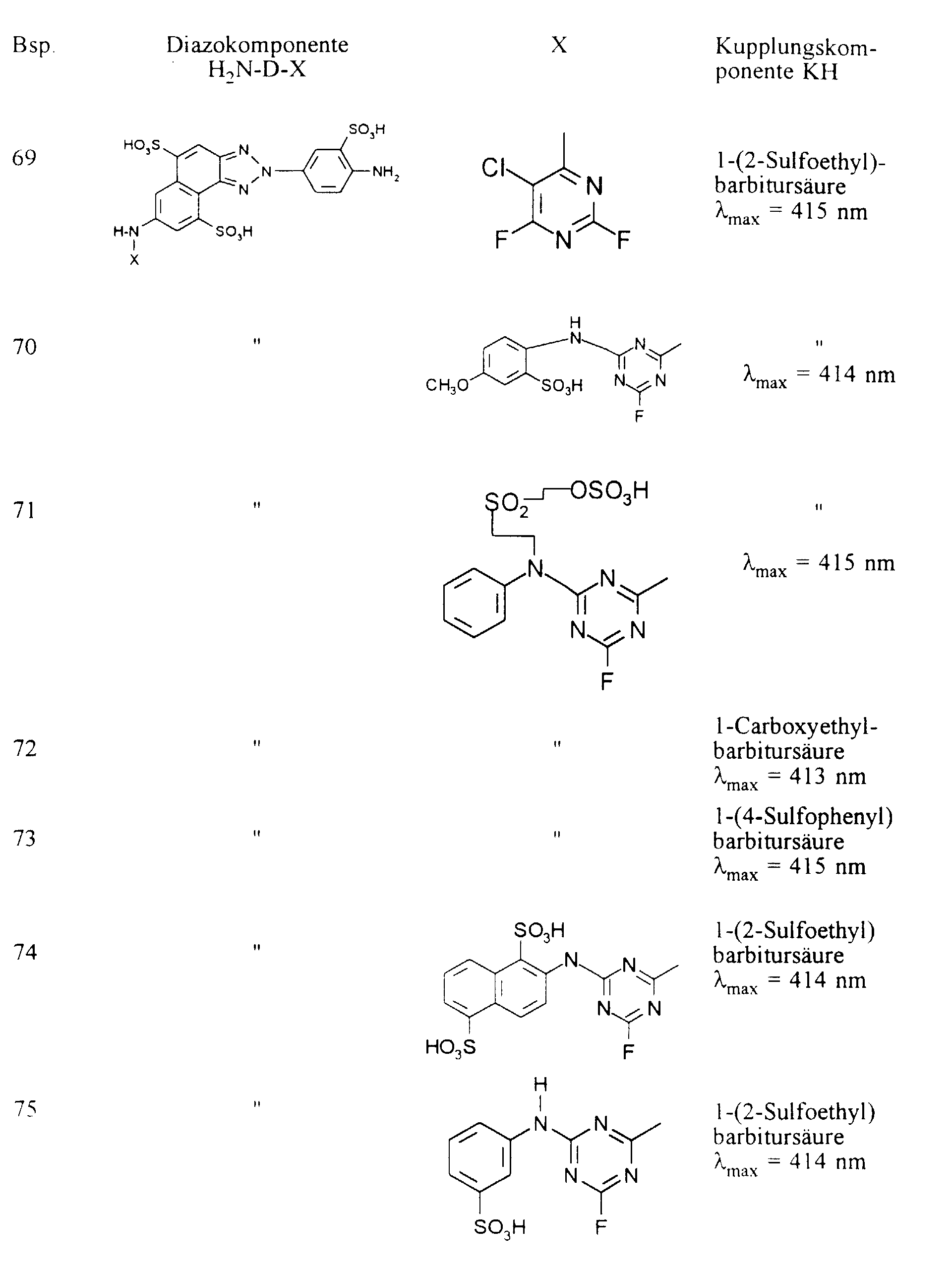

### Beispiel 76

0,32 Mol 2,4,6-Trichlor-1,3,5-triazin werden bei 0°C in 200 ml Wasser suspendiert. Zu dieser Mischung wird in 20 Minuten eine neutrale Lösung von 0,3 Mol 3-Amino-4-methoxybenzolsulfonsäure in 1 l Wasser gegeben. Mit Sodalösung wird ein pH-Wert von 4,5 bis 5,0 gehalten. Es wird 1 Stunde bei 0 bis 5°C nachgerührt. Überschüssiges 2,4,6-Trichlor-1,3,5-triazin wird abfiltriert.

Zu einer neutralen Lösung von 0,29 Mol des Triazols der Formel 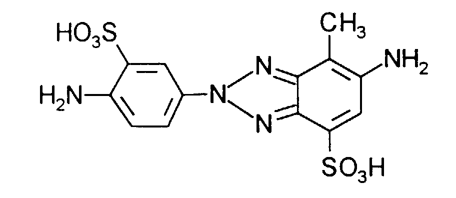 in 2,5 l Wasser werden 120 g Natriumhydrogencarbonat gegeben. Nach Abkühlen der Lösung auf 15°C wird obige Mischung in 10 bis 15 Minuten zugegeben. Es wird 1 Stunde bei 40°C nachgerührt. Nach Abkühlen der Mischung auf 0 bis 5°C und Zugabe von 70 ml 30%iger Natriumnitritlösung wird mit 30%iger Salzsäure ein pH-Wert von 2,8 bis 3,0 eingestellt.

Es wird 30 Minuten nachgerührt. Nach Zerstören des Natriumnitritüberschüsses mit Amidosulfonsäure werden 0,3 Mol 1-(2'-Sulfoethyl)-barbitursäure in die Diazotierung gegeben. Mit Sodalösung wird ein pH-Wert von 6,5 eingestellt. Es wird 1 Stunde bei Raumtemperatur und pH = 6,5 nachgerührt. Der nach Aussalzen, Isolierung, Trocknung und Mahlen erhaltene Farbstoff der Formel 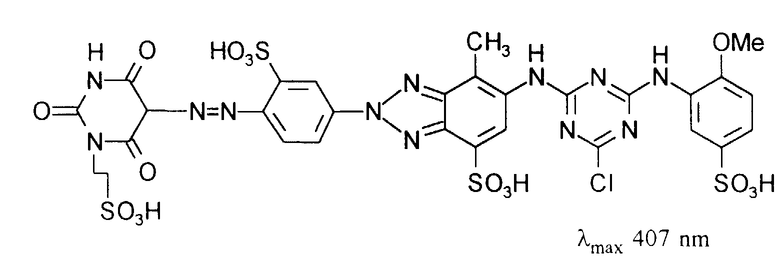 färbt Baumwolle in grünstichig gelben Tönen.

Weitere entsprechende Farbstoffe K-N=N-D-X sind:

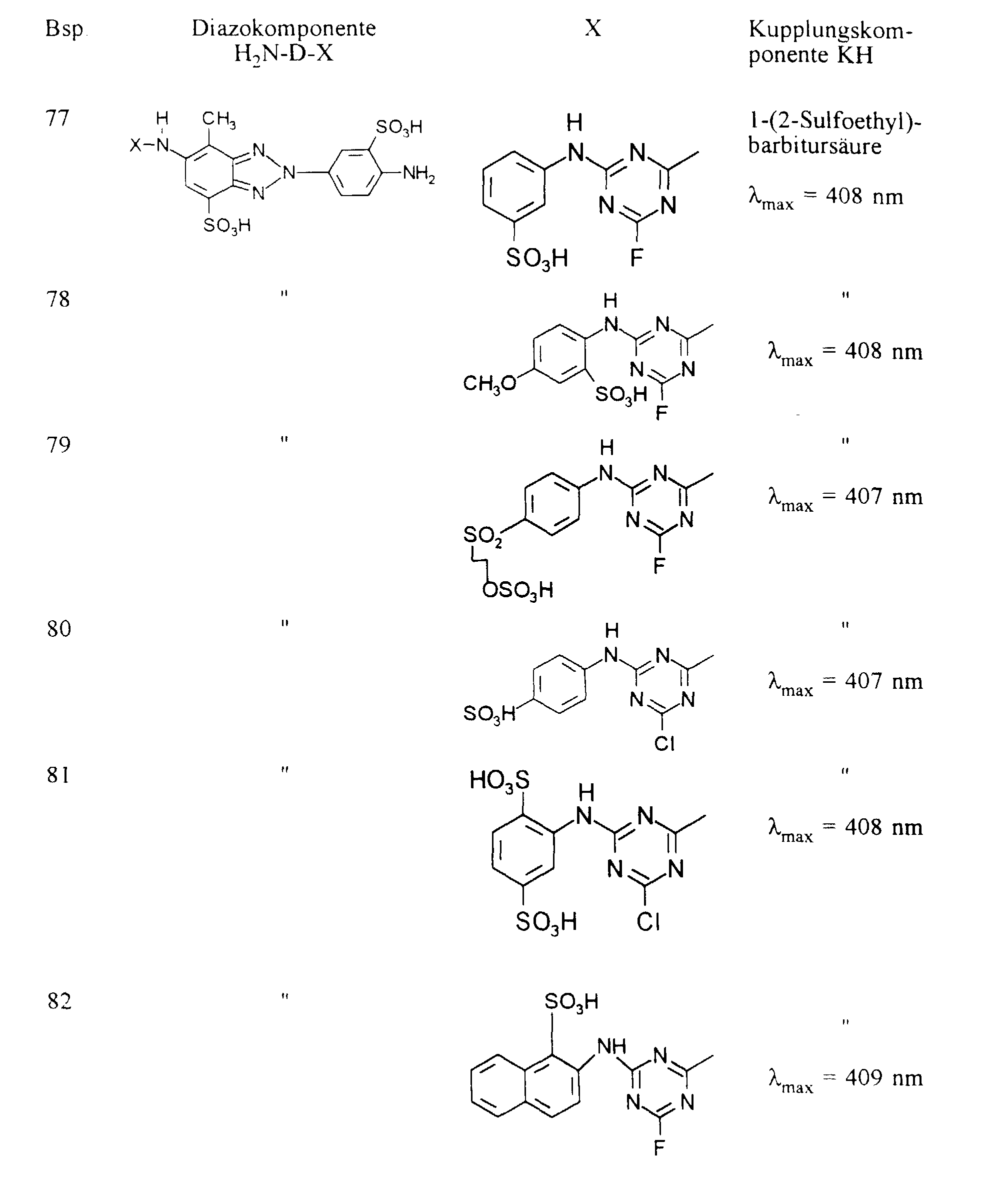

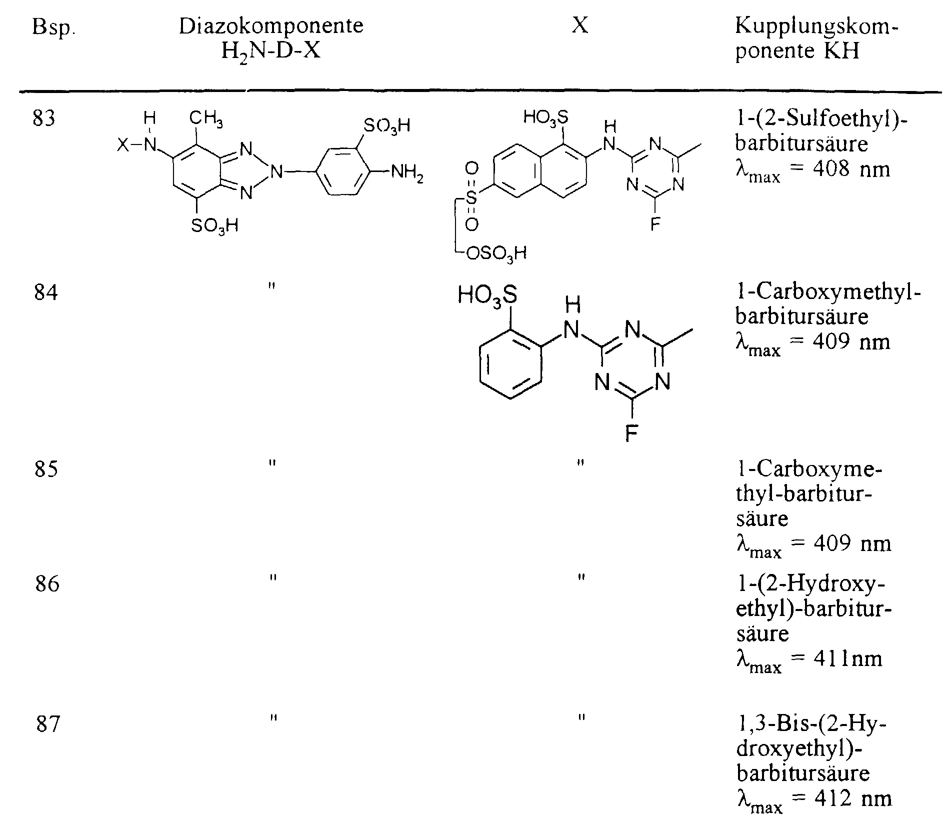

### Beispiel 88

Einer Lösung von 0,29 Mol des Triazols der Formel 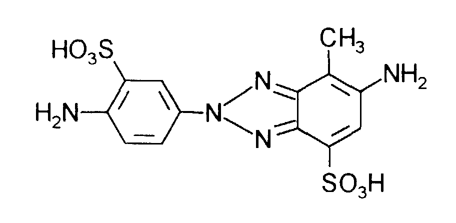 in 3 l Wasser werden bei 0°C und pH = 4 bis 4,5 0,334 Mol 2,4,6-Trifluor-1,3,5-triazin zugesetzt. Nach 10 Minuten werden 0,318 Mol 3-Aminobenzyl-β-sulfatoethylsulfon zugegeben. Mit Sodalösung wird ein pH-Wert von 7 bis 7,5 eingestellt und gehalten. Es wird 2 Stunden bei Raumtemperatur nachgerührt. Anschließend wird bei 0 bis 5°C und pH = 2,8 bis 3,0 diazotiert. Nach Zerstören des Nitritüberschusses werden 0,3 Mol 1,3-Bis-(2-hydroxypropyl)barbitursäure in die Diazotierung gegeben. Mit Sodalösung wird ein pH-Wert von 6,5 bis 7,0 eingestellt. Der nach Aussalzen, Isolierung, Trocknung und Mahlen erhaltene Farbstoff der Formel 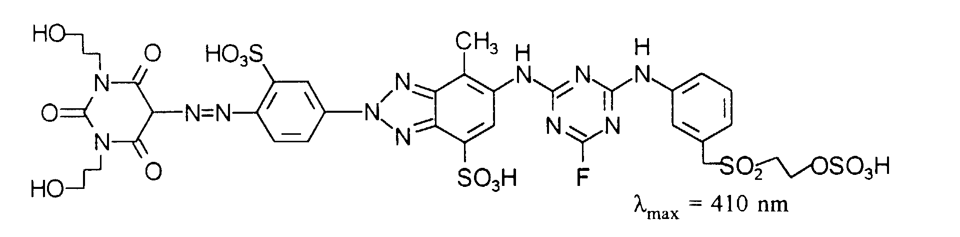 färbt Baumwolle in grünstichig gelben Tönen.

### Beispiel 89

Eine Lösung von 0,29 Mol des Triazols der Formel 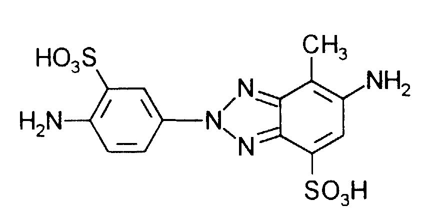 in 3 l Wasser werden bei 0°C und pH = 4 bis 4,5 0,334 Mol 2,4,6-Trifluor-1,3,5-triazin zugesetzt. Nach 10 Minuten werden 0,318 Mol Morpholin zugegeben. Mit Sodalösung wird ein pH-Wert von 7 bis 7,5 eingestellt und gehalten. Es wird 2 Stunden bei Raumtemperatur nachgerührt. Anschließend wird bei 0 bis 5°C und pH = 2,8 bis 3,0 diazotiert. Nach Zerstören des Nitritüberschusses werden 0,3 Mol 1-(2-Sulfoethyl)barbitursäure in die Diazotierung gegeben. Mit Sodalösung wird ein pH-Wert von 6,5 bis 7,0 eingestellt. Der nach Aussalzen, Isolierung, Trocknung und Mahlen erhaltene Farbstoff der Formel 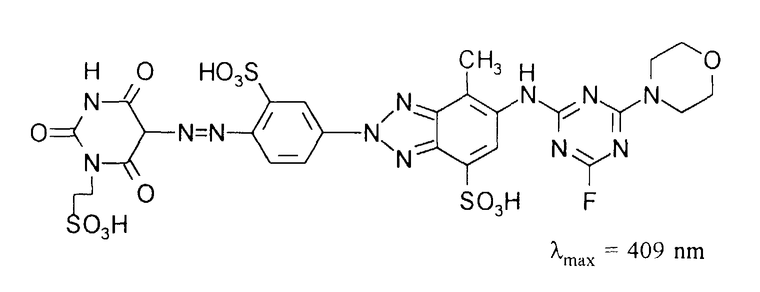 färbt Baumwolle in grünstichig gelben Tönen mit hohem Echtheitsniveau. Dieser wertvolle Farbstoff ist alternativ auch folgendermaßen herstellbar:

### Beispiel 90

0,29 Mol des Triazols der Formel 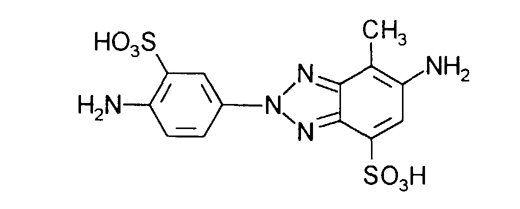 werden in 2 l Wasser bei pH = 6,0 suspendiert. Bei 5°C werden in die Suspension in 60 Minuten 0,45 Mol Maleinsäureanhydrid portionsweise eingetragen, dabei wird mit 20%iger Natronlauge der pH-Wert bei 6,0 gehalten. Es wird 30 Minuten nachgerührt. 70 ml 30%ige Natriumnitritlösung werden zur Lösung gegeben. Mit Salzsäure wird ein pH-Wert von 2,0 bis 2,5 eingestellt. Die Diazotierung wird bei 5 bis 10°C 45 Minuten nachgerührt. Nach Zerstören des Nitritüberschusses mit Amidosulfonsäure werden 0,3 Mol 1-(2-Sulfoethyl)barbitursäure in die Diazotierung gegeben. Mit Sodalösung wird ein pH-Wert von 6,5 bis 7,0 eingestellt. Es wird 15 Minuten bei Raumtemperatur nachgerührt. Anschließend wird die Mischung auf 80°C erwärmt. Mit konzentrierter Salzsäure wird ein pH-Wert von 0,5 bis 1,0 eingestellt. Nach 2 Stunden läßt man die Mischung abkühlen. Der gebildete orange Niederschlag wird abgesaugt und in 7 l Wasser bei pH = 7,0 und 60°C gelöst. Anschließend wird schnell auf 0°C abgekühlt. Bei pH = 4 bis 4,5 werden 0,32 Mol 2,4,6-Trifluor-1,3,5-triazin zugegeben. Nach 10 Minuten werden 0,3 Mol Morpholin zugetropft.

Mit Sodalösung wird ein pH-Wert von 7,0 bis 7,5 eingestellt und gehalten. Es wird 2 Stunden bei Raumtemperatur nachgerührt. Der nach Aussalzen, Isolierung, Trocknung und Mahlen erhaltene Farbstoff der Formel 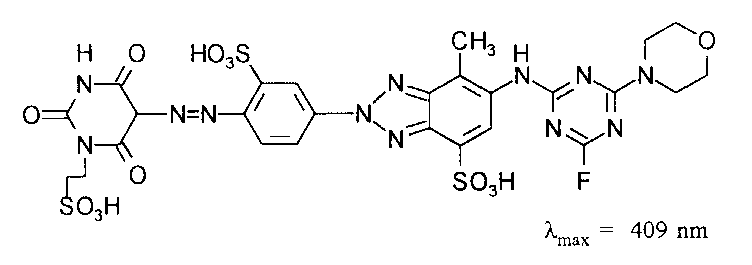 färbt Baumwolle in grünstichig gelben Tönen mit hohem Echtheitsniveau.

Nach dieser oder der vorhergehenden Vorschrift sind weitere interessante Reaktivfarbstoffe K-N=N-D-X zugänglich:

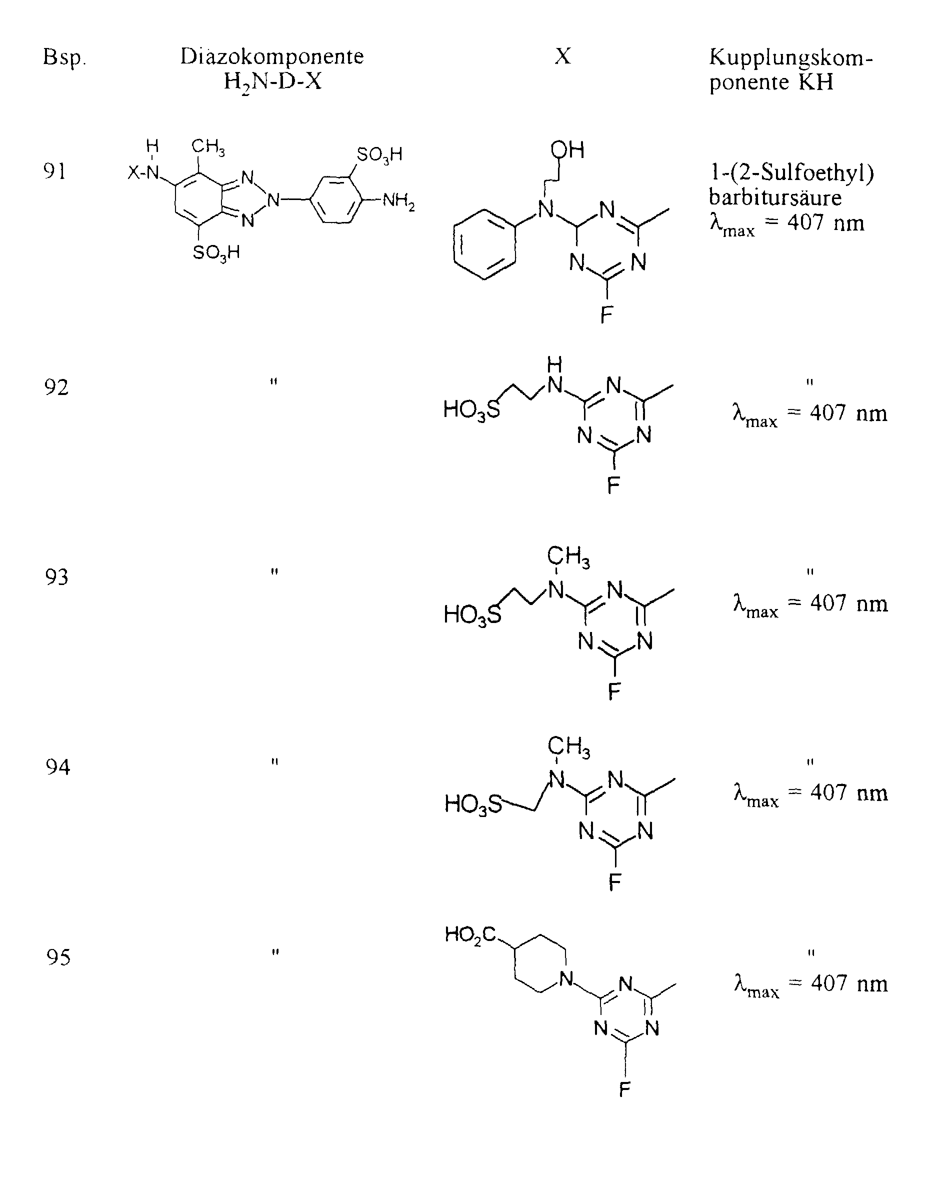

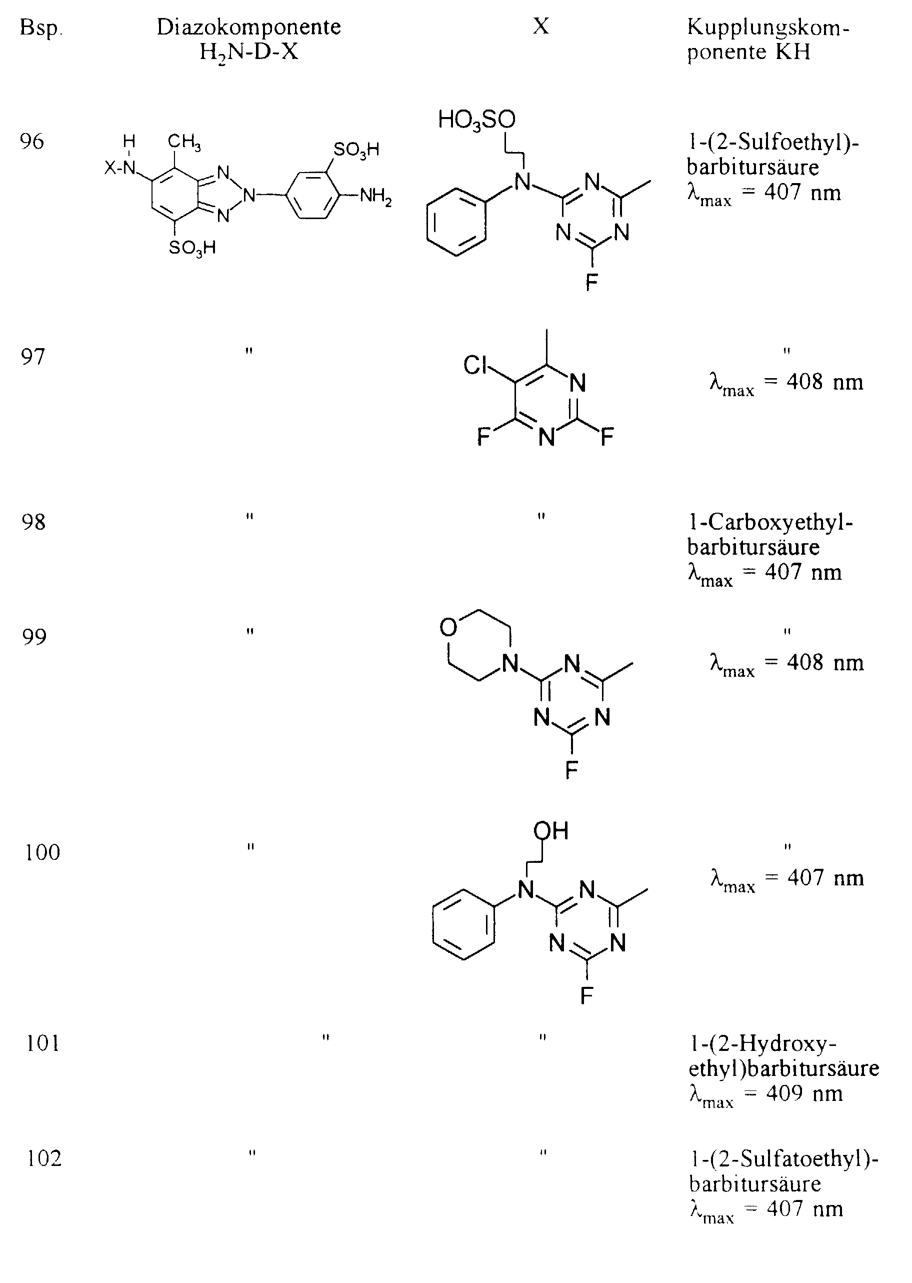

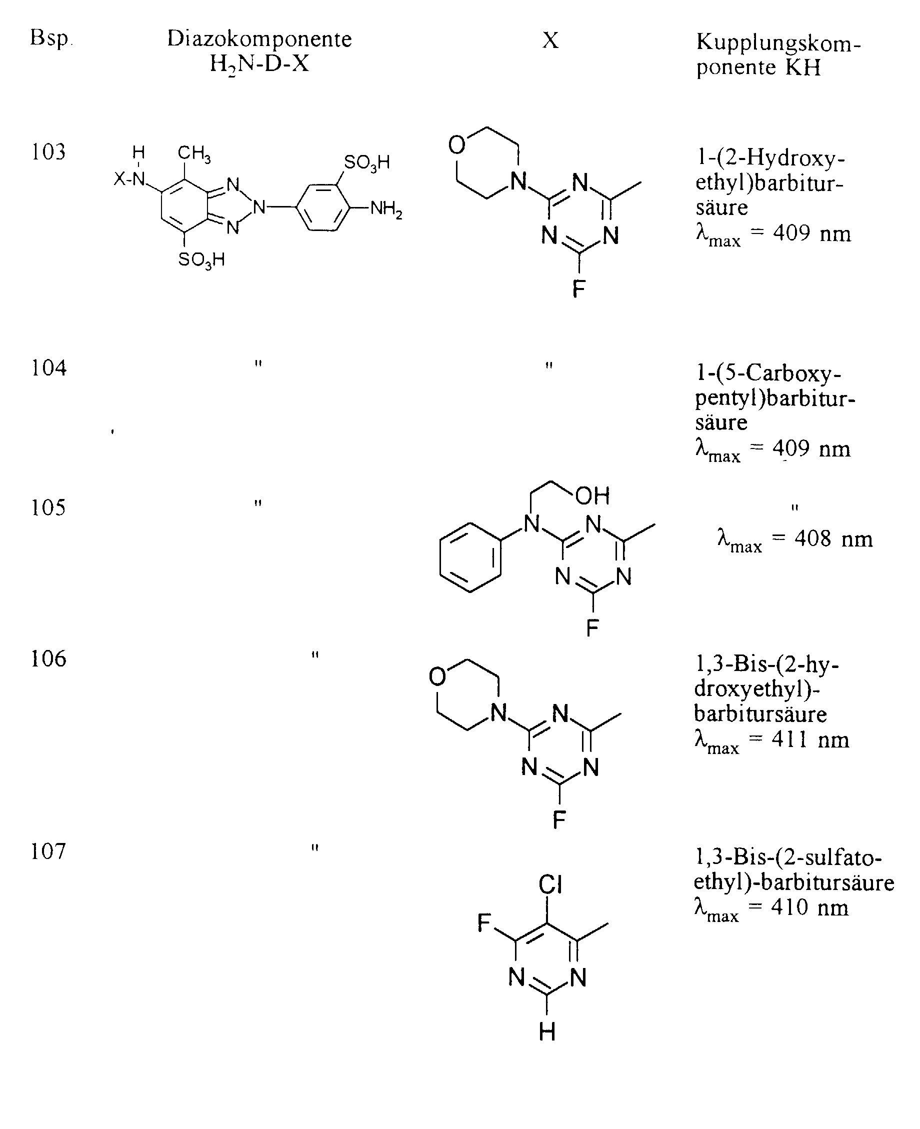

## Patentansprüche

1. Faserreaktiver Azofarbstoff der Formel (1) und dessen Tautomere 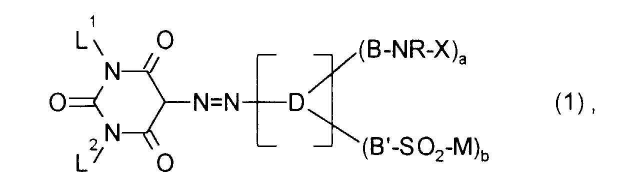 worin bedeuten:
a = 0, 1 oder 2,
b = 0, 1 oder 2,
a + b = 1 oder 2
D = Rest einer aromatischen oder heterocyclischen Diazokomponente,
B,B' = unabhängig voneinander direkte Bindung oder Brückenglied,
X = faserreaktiver heterocyclischer Rest,
R = H, gegebenenfalls substituiertes C₁-C₄-Alkyl,
M = CH₂-CH₂-OH, CH=CH₂ oder CH₂-CH₂-V, worin V = alkalisch eliminierbarer Rest,
L¹, L² = gleich oder verschieden, H oder eine aliphatische oder aromatische Gruppe wobei wenigstens einer der Substituenten L¹ und L² mit wenigstens einem polaren Rest substituiert ist oder für Hydroxy steht.

2. Farbstoff gemäß Anspruch 1, worin bedeuten
L¹ = H, 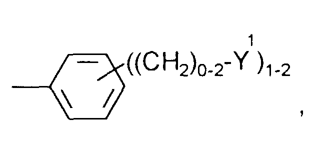 -(CH₂)₁₋₅-Y²,
L² = 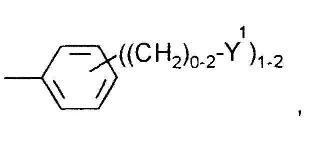 -(CH₂)₁₋₅-Y², -OH,
Y¹ = unabhängig voneinander bei L¹ und L²:
OH, COOH, SO₃H, C₁-C₄-Alkoxy, Hydroxy-C₁₋₄-Alkoxy-, HO-(CH₂-CH₂-O)₁₋₃-, HOOC-(CH₂-CH₂-O)₁₋₃-, HO₃S-(CH₂-CH₂-O)₁₋₃-, HO₃S-CH₂-CH₂-CH₂-O-, HOOC-CH₂-CH₂-CH₂-O-,
Y² = unabhängig voneinander bei L¹ und L²:
OH, COOH, OSO₃H, SO₃H, C₁-C₄-Alkoxy, Hydroxy-C₁₋₄-Alkoxy, HO-(CH₂-CH₂-O)₁₋₃-, HOOC-(CH₂-CH₂-O)₁₋₃-, HO₃S-(CH₂-CH₂-O)₁₋₃-, HO₃S-CH₂-CH₂-CH₂-O-, HOOC-CH₂-CH₂-CH₂-O-,

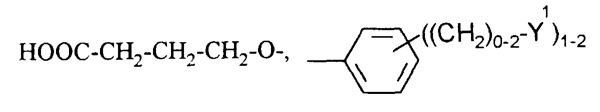

3. Farbstoff der Formel (1) gemäß Anspruch 1, worin
a = 0 und
-D-(B'-SO₂-M)_{b} einem Rest folgender Formel (3) entspricht 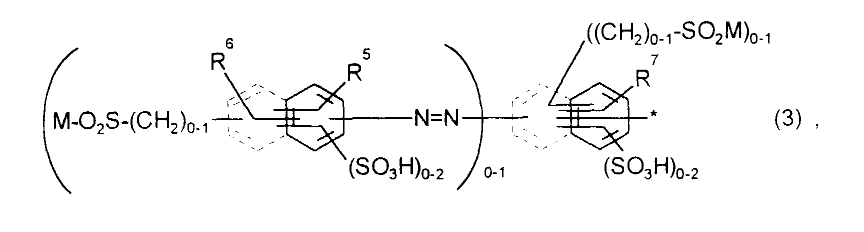 worin bedeuten
R⁵ H, C₁-C₄-Alkyl, Cl, Br, C₁-C₄-Alkoxy oder COOH,
R⁶ H, C₁-C₄-Alkyl, C₁-C₄-Alkoxy, SO₃H, Cl oder Br,
R⁷ H, C₁-C₄-Alkyl, C₁-C₄-Alkoxy, Cl, Br, Acylamino, insbesondere C₁-C₄-Alkylcarbonylamino oder Arylcarbonylamino, wie gegebenenfalls substituiertes Phenylcarbonylamino, C₁-C₄-Alkylsulfonylamino, Aminocarbonylamino, Arylsulfonylamino,
und die mit * gekennzeichnete Bindung an die Azogruppe der Formel (1) gebunden ist.

4. Farbstoff der Formel (1) gemäß Anspruch 1, worin
b = 0 und
a 1 bedeutet.

5. Farbstoff gemäß Anspruch 1 der Formel (4) 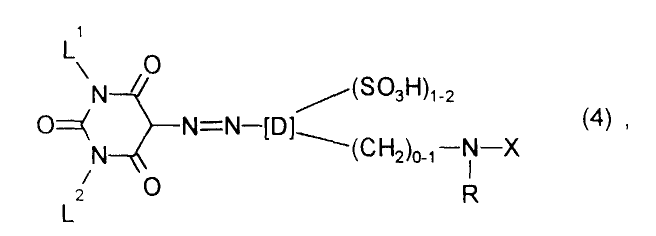 oder der Formel (5) 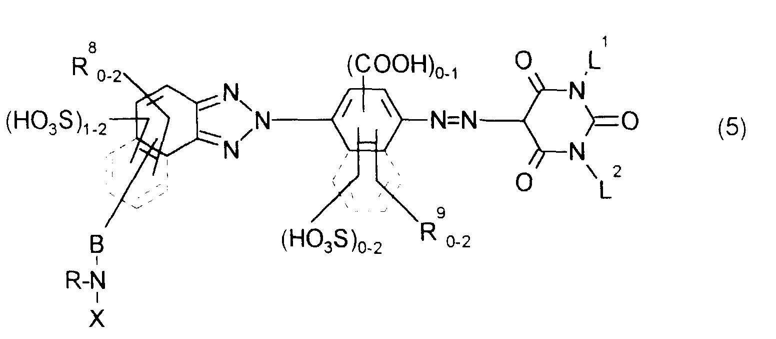 oder der Formel (6) 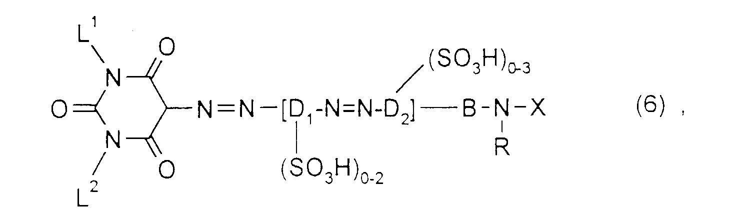 worin
oder der Formel (7) 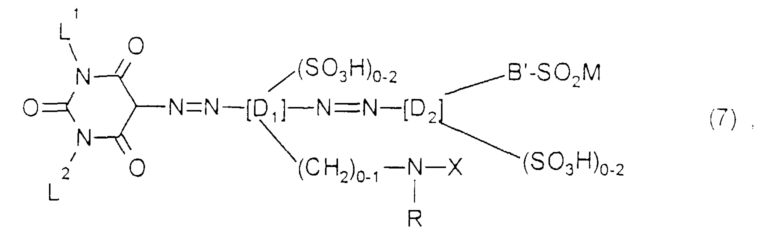 worin
D = Rest eines Benzols oder Naphthalins, das gegebenenfalls durch C₁-C₄-Alkyl, C₁-C₄-Alkoxy, CO₂H, 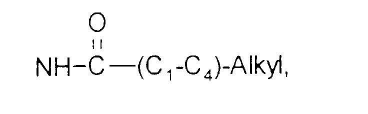 NH-CO-NH₂ oder Halogen substituiert ist,
R⁸ und R⁹ unabhängig voneinander für Wasserstoff, C₁-C₄-Alkyl, C₁-C₄-Alkoxy, Acetylamino, NH₂, Methylamino, Ethylamino, Carbamoyl, Ureido, Hydroxy oder Acetyl stehen,
D₁ und D₂ unabhangig voneinander einen gegebenenfalls durch C₁-C₄-Alkyl, C₁-C₄-Alkoxy, CO₂H, NHCO-C₁-C₄-Alkyl, NHCONH₂ oder Halogen substituiertes Phenylen- oder Naphthylenrest bedeuten und und
L¹, L², M, B', R und X die Bedeutungen gemäß Anspruch 1 besitzen.

6. Farbstoff gemäß Anspruch 1, dadurch gekennzeichnet, daß
B und B' unabhängig voneinander eine direkte Bindung oder ein Brückenglied der Formeln 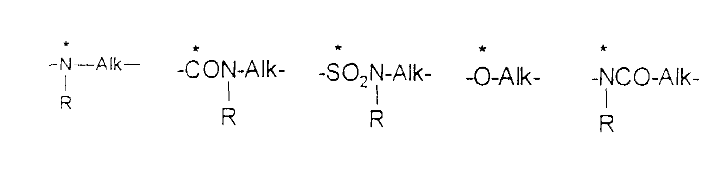
X für 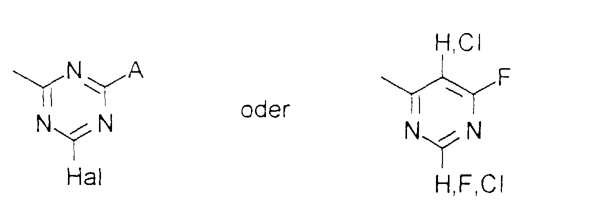
steht, worin
Hal = Cl oder F und
A = Rest eines Amins AH.

7. Verfahren zur Herstellung der Farbstoffe gemäß Anspruch 1, dadurch gekennzeichnet, daß man Verbindungen der Formel (10) 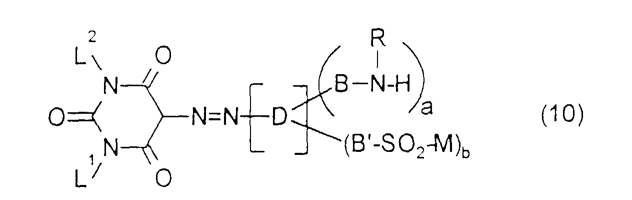 bzw. die entsprechenden Diazokomponenten der Formel (11) 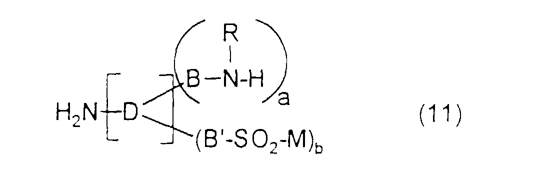 mit 1 bis 2 Mol-Äquivalenten einer Reaktivkomponente der Formel
X-Hal,
umsetzt, wobei X einen faserreaktiven heterocyclischen Rest bedeutet und Hal für Halogen steht, und im Falle der Verwendung von Vorprodukten diese anschließend in die gewünschten Endfarbstoffe überführt und gegebenenfalls weitere Umwandlungsreaktionen anschließt.

8. Verbindungen der Formel (12) 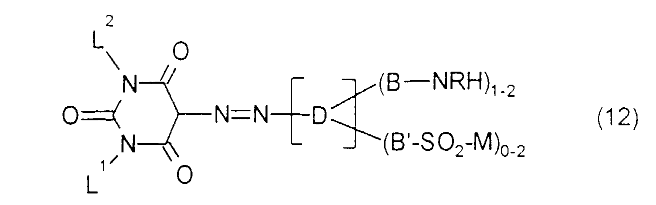 worin L¹, L², B, B', R, D und M die in Anspruch 1 angegebene Bedeutung haben.

9. Cellulose- oder polyamidhaltige Materialien, die mit einem Reaktivfarbstoff gemäß Anspruch 1 gefärbt sind.

## Claims

1. Fibre-reactive azo dyestuff of the formula (1) and tautomers thereof 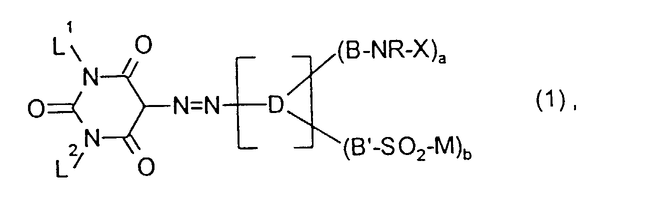 wherein
a = 0, 1 or 2,
b = 0, 1 or 2,
a + b= 1 or 2,
D = the radical of an aromatic or heterocyclic diazo component,
B and B' = independently of one another a direct bond or bridge member,
X = a fibre-reactive heterocyclic radical,
R = H or optionally substituted C₁-C₄-alkyl,
M = CH₂-CH₂-OH, CH=CH₂ or CH₂-CH₂-V, wherein V = a radical which can be eliminated under alkaline conditions, and
L¹ and L² = as identical or different radicals, H or an aliphatic or aromatic group, wherein at least one of the substituents L¹ and L² is substituted by at least one polar radical or represents hydroxyl.

2. Dyestuff according to Claim 1, wherein
L¹ = 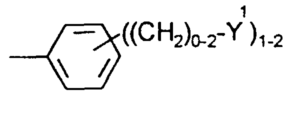 or -(CH₂)₁₋₅-Y²,
L² = 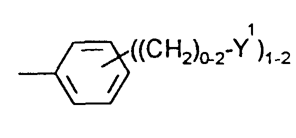 or -(CH₂)₁₋₅-Y², -OH,
Y¹ = independently of one another in L¹ and L²:
OH, COOH, SO₃H, C₁-C₄-alkoxy, hydroxy-C₁₋₄-alkoxy, HO-(CH₂-CH₂-O)₁₋₃-, HOOC-(CH₂-CH₂-O)₁₋₃-, HO₃S-(CH₂-CH₂-O)₁₋₃-, HO₃S-CH₂-CH₂-CH₂-O-, or HOOC-CH₂-CH₂-CH₂-O-, and
Y² = independently of one another in L¹ and L²:
OH, COOH, OSO₃H, SO₃H, C₁-C₄-alkoxy, hydroxy-C₁₋₄-alkoxy, HO-(CH₂-CH₂-O)₁₋₃-, HOOC-(CH₂-CH₂-O)₁₋₃-, HO₃S-(CH₂-CH₂-O)₁₋₃-, HO₃S-CH₂-CH₂-CH₂-O-, HOOC-CH₂- CH₂-CH₂-O- or

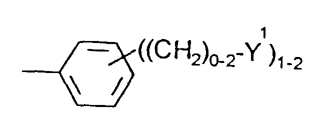

3. Dyestuff of the formula (1) according to Claim 1, wherein
a = 0, and
-D-(B'-SO₂-M)_{b} corresponds to a radical of the following formula (3) 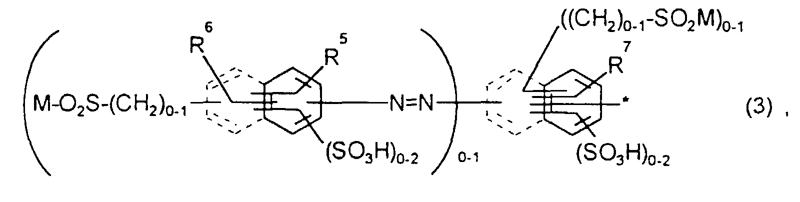 wherein
R⁵ denotes H, C₁-C₄-alkyl, Cl, Br, C₁-C₄-alkoxy or COOH,
R⁶ denotes H, C₁-C₄-alkyl, C₁-C₄-alkoxy, SO₃H, Cl or Br,
R⁷ denotes H, C₁-C₄-alkyl, C₁-C₄-alkoxy, Cl, Br, acylamino, in particular C₁-C₄-alkylcarbonylamino or arylcarbonylamino, such as optionally substituted phenylcarbonylamino, C₁-C₄-alkylsulphonylamino, aminocarbonylamino or arylsulphonylamino,
and the bond identified with ^{*} is bonded to the azo group of the formula (1).

4. Dyestuff of the formula (1) according to Claim 1, wherein
b = 0 and
a = 1.

5. Dyestuff according to Claim 1 of the formula (4) 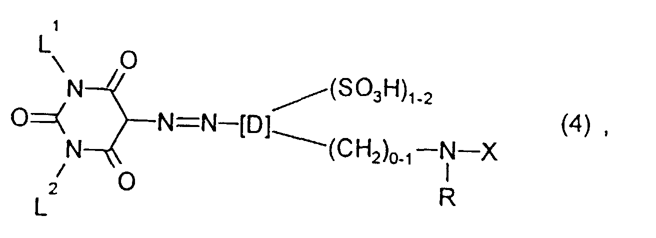 or of the formula (5) 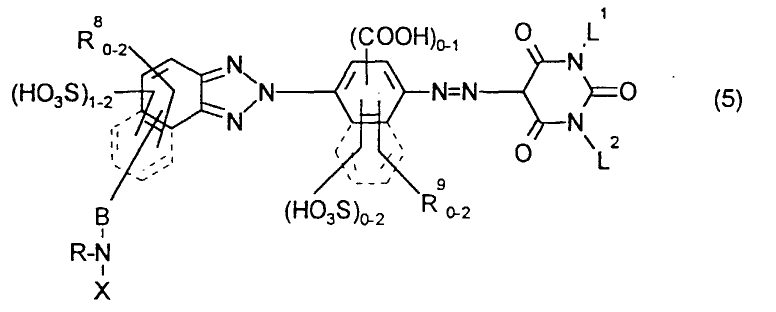 or of the formula (6) 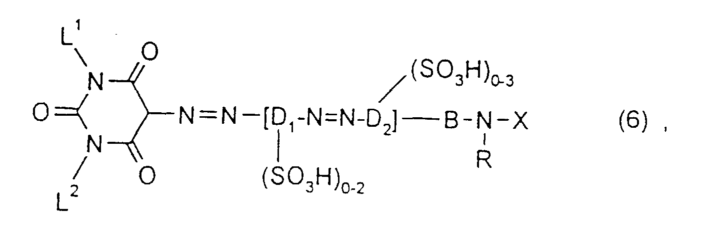 the formula (7) 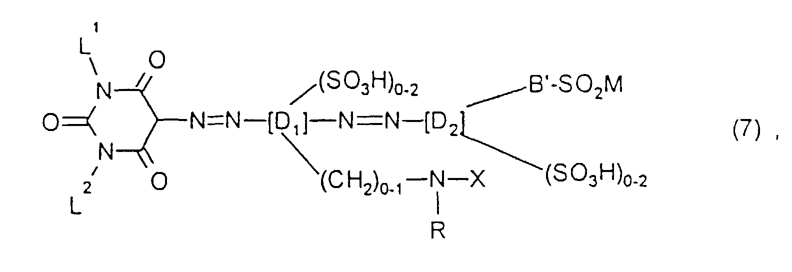 wherein
D = the radical of a benzene or naphthalene, which is optionally substituted by C₁-C₄-alkyl, C₁-C₄-alkoxy, CO₂H, 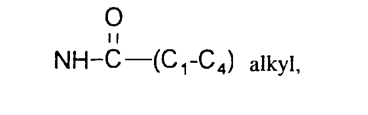 NH-CO-NH₂ or halogen,
R⁸ and R⁹ independently of one another represent hydrogen, C₁-C₄-alkyl, C₁-C₄-alkoxy, acetylamino, NH₂, methylamino, ethylamino, carbamoyl, ureido, hydroxyl or acetyl,
D₁ and D₂ independently of one another denote a phenylene or naphthylene radical, which is optionally substituted by C₁-C₄-alkyl, C₁-C₄-alkoxy, CO₂H, NHCO-C₁-C₄-alkyl, NHCONH₂ or halogen, and
L¹, L², M, B', R and X have the meanings given in Claim 1.

6. Dyestuff according to Claim 1, characterized in that
B and B' independently of one another denote a direct bond or a bridge member of the formulae 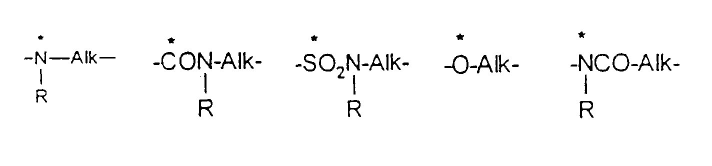
X represents 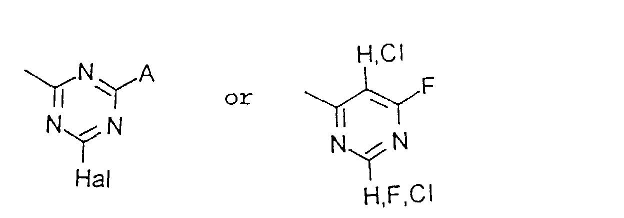 wherein
Hal = Cl or F and
A = the radical of an amine AH.

7. Process for the preparation of the dyestuffs according to Claim 1, characterized in that compounds of the formula (10) 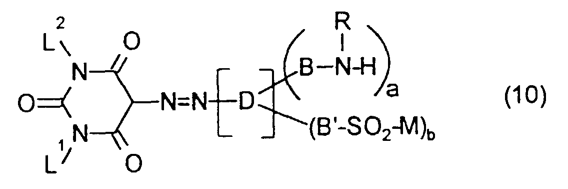 or the corresponding diazo components of the formula (11) 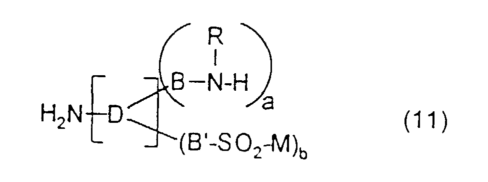 are reacted with 1 to 2 molar equivalents of a reactive component of the formula
X-Hal,
wherein X denotes a fibre-reactive heterocyclic radical and Hal represents halogen, and, if precursors are used, these are then converted into the desired end dyestuffs, and if appropriate further conversion reactions are subsequently carried out.

8. Compounds of the formula (12) 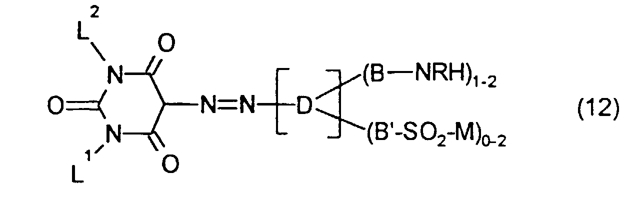 wherein L¹, L², B, B', R, D and M have the meanings given in Claim 1.

9. Materials containing cellulose or polyamide which are dyed with a reactive dyestuff according to Claim 1.

## Revendications

1. Colorant azo pouvant réagir avec des fibres de la formule (I) et tautomères de celui-ci 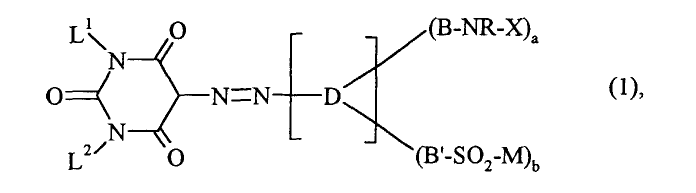 dans laquelle
a = 0, 1 ou 2
b = 0, 1 ou 2
a+b = 1 ou 2
D = reste d'un constituant diazo aromatique ou hétérocyclique,
B,B' = indépendamment une liaison directe ou un élément de pontage
X = reste hétérocyclique pouvant réagir avec des fibres,
R= H, éventuellement un groupe alkyle en C₁-C₄ substitué;
M= CH₂-CH₂-OH, CH=CH₂ ou CH₂-CH₂-V, V= reste pouvant être éliminé par un alcali,
L¹,L² = de manière identique ou différente, H ou un groupe aliphatique ou aromatique, au moins un des substituants L¹ et L² étant substitué avec au moins un reste polaire ou étant un groupe hydroxy.

2. Colorant selon la revendication 1, dans lequel
L¹ = H, 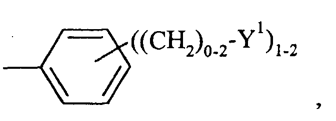 -(CH₂)₁₋₅-Y²,
L² = 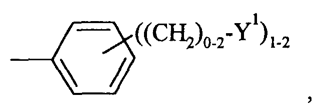 -(CH₂)₁₋₅-Y², -OH,
Y¹ = indépendamment pour L¹ et L² :
OH, COOH, SO₃H, groupe alcoxy en C₁-C₄, groupe hydroxy-alcoxy en C₁-C₄, HO-(CH₂-CH₂-O)₁₋₃-, HOOC-(CH₂-CH₂-O)₁₋₃-, HO₃S-(CH₂-CH₂-O)₁₋₃-, HO₃S-CH₂-CH₂-CH₂-O-, HOOC-CH₂-CH₂-CH₂-O-,
Y² = indépendamment pour L¹ et L² :
OH, COOH, OSO₃H, SO₃H, groupe alcoxy en C₁-C₄, groupe hydroxy-alcoxy en C₁-C₄, HO-(CH₂-CH₂-O)₁₋₃-, HOOC-(CH₂-CH₂-O)₁₋₃-, HO₃S-(CH₂-CH₂-O)₁₋₃-, HO₃S-CH₂-CH₂-CH₂-O-, HOOC-CH₂-CH₂-CH₂-O-,

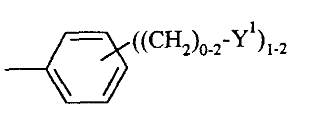

3. Colorant de la formule (1) selon la revendication 1, dans lequel
a = 0 et
-D-(B'-SO₂-M)_{b} correspond à un reste de la formule (3) suivante 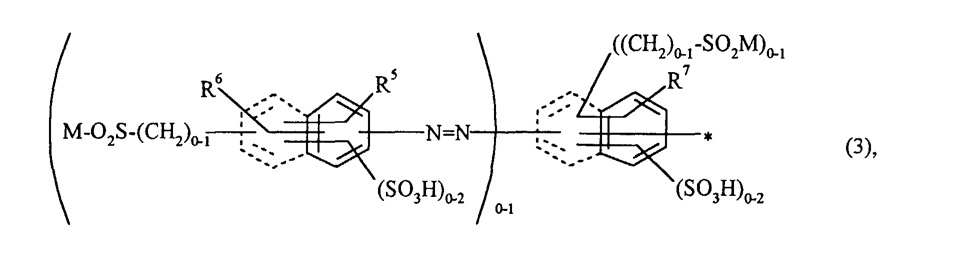 dans laquelle
R⁵ représente H, un groupe alkyle en C₁-C₄, Cl, Br, un groupe alcoxy en C₁-C₄ ou COOH,
R⁶ représente H, un groupe alkyle en C₁-C₄, un groupe alcoxy en C₁-C₄, SO₃H, Cl ou Br,
R⁷ représente H, un groupe alkyle en C₁-C₄, un groupe alcoxy en C₁-C₄, Cl, Br, un groupe acylamino, en particulier un groupe alkylcarbonylamino en C₁-C₄ ou arylcarbonylamino, comme un groupe phénylcarbonylamino éventuellement substitué, alkylsulfonylamino en C₁-C₄, aminocarbonylamino, arylsulfonylamino,
et la liaison caractérisée par * est liée au groupe azo de la formule (1).

4. Colorant de la formule (1) selon la revendication 1, dans lequel
b = 0 et
a = 1

5. Colorant selon la revendication 1 de la formule (4) 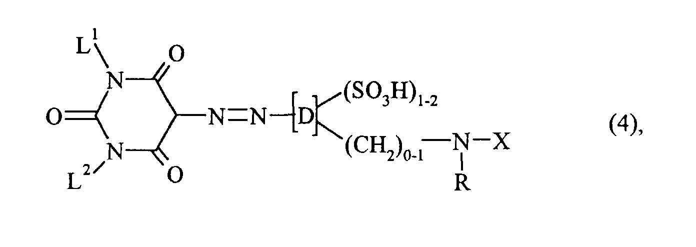 ou de la formule (5) 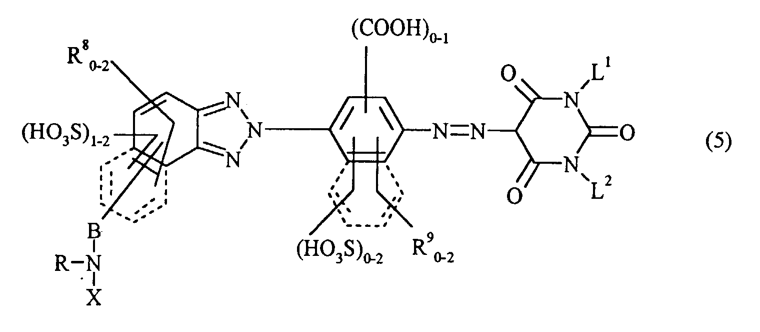 ou de la formule (6) 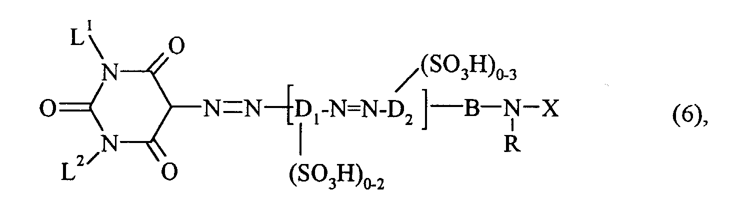 ou de la formule (7) 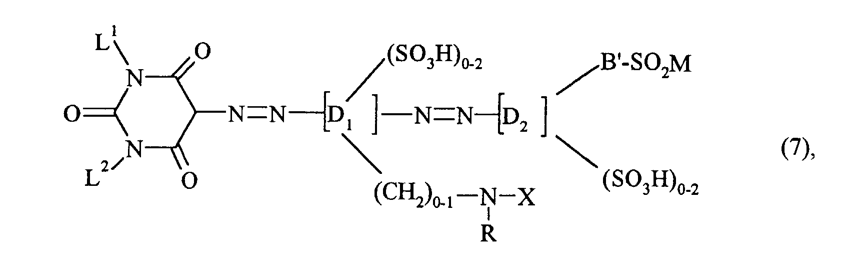 dans laquelle
D = Reste d'un benzène ou d'un naphtalène, qui est éventuellement substitué par un groupe alkyle en C₁-C₄, alcoxy en C₁-C₄, CO₂H, 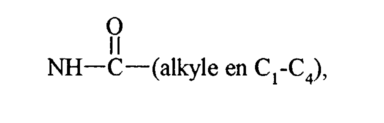
NH-CO-NH₂ ou un halogène,
R⁸ et R⁹ représentent indépendamment l'hydrogène, un groupe alkyle en C₁-C₄, alcoxy en C₁-C₄, acétylamino, NH₂, méthylamino, éthylamino, carbamoyle, uréido, hydroxy ou acétyle,
D₁ et D₂ représentent indépendamment un reste phénylène ou naphtylène éventuellement substitué par un groupe alkyle en C₁-C₄, alcoxy en C₁-C₄, CO₂H, NHCO-(alkyle en C₁-C₄), NHCONH₂ ou un halogène et
L¹, L², M, B', R et X ont les significations indiquées dans la revendication 1.

6. Colorant selon la revendication 1, caractérisé en ce que B et B' représentent indépendamment une liaison directe ou un élément de pontage des formules 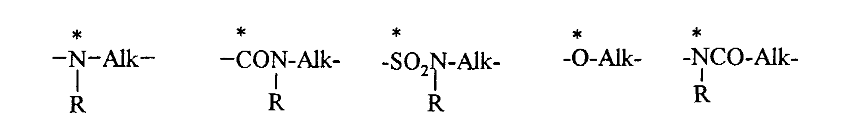
X représente 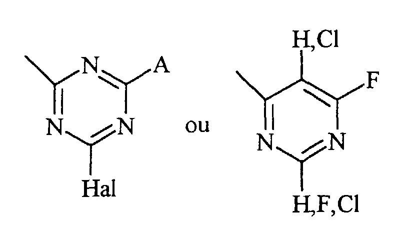 où
Hal = Cl ou F et
A = Reste d'une amine AH.

7. Procédé pour la préparation du colorant selon la revendication 1, caractérisé en ce que l'on fait réagir des composés de la formule (10) 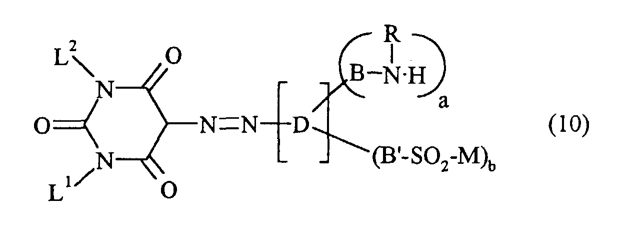 respectivement les constituants diazo correspondants de la formule (11) 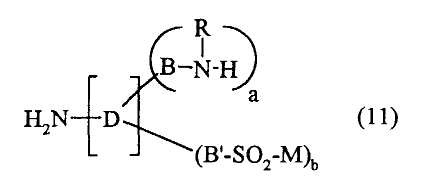 avec de 1 à 2 équivalents en mol d'un constituant réactif de la formule
X-Hal,
X représentant un reste hétérocyclique pouvant réagir avec des fibres et Hal représentant un halogène, et en ce que dans le cas de l'utilisation de pré-produits on transforme ceux-ci ensuite en les colorants finaux souhaités et on les soumet ensuite éventuellement à d'autres réactions de transformation.

8. Composés de la formule (12) 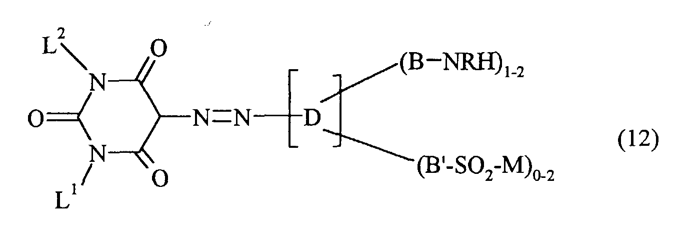 dans laquelle L¹, L², B, B', R, D et M ont les significations indiquées dans la revendication 1.

9. Matériaux contenant de la cellulose ou du polyamide, lesquels sont colorés avec un colorant réactif selon la revendication 1.
